# EUROPEAN PATENT APPLICATION

(11) **EP 1 452 184 A1**
(43) Date of publication of application: **01.09.2004**
(21) Application number: 03004168.5
(22) Date of filing: 26.02.2003
(51) Int. Cl.: A61K 38/17, A61P 33/06

(54) **Use of thioester-containing proteins (TEPs) for triggering/inducing an immune response in mosquitoes of Anopheles against plasmodium malariae**

(71) Applicant: EMBL, D-69117 Heidelberg (DE)
(72) Inventor: Levashina, Elena, 67000 Strasbourg (FR); Blandin, Stephanie, 69126 Heidelberg (DE); Kafatos, Fotis, 69120 Heidelberg (DE); Clayton, John Randall, Baltimore, MD 21202 (US); Shiao, Shin-Hong, 67000 Strasbourg (FR); Moita, Luis Cent. for Immun. and Inflam. Diseases, Charlestown, MA 02129 (US)
(74) Representative: Krauss, Jan B., Dr.

(57) **Abstract**

The present invention relates to the prevention of malaria transmission. It focuses on the use of thioester-containing proteins that function in the immune system of the mosquito Anopheles gambiae which is the major vector of human malaria. The invention furthermore relates to a method of making mosquitoes of the genus Anopheles refractory/susceptible towards the malaria parasite plasmodium, and to refractory/susceptible mosquitoes produced according to this method.

SEQUENCE LISTING

<110>  EMBL

<120>  Use of thioester-containing proteins (TEPs) for
triggering/inducing an immune response in mosquitoes of Anopheles
against plasmodium

<130>  FB12312

<160>  10

<170>  PatentIn version 3.1

<210>  1
<211>  1340
<212>  PRT
<213>  Anopheles gambiae

<220>
<221>  MISC_FEATURE
<222>  (90)..(90)
<223>  any amino acid residue


<220>
<221>  MISC_FEATURE
<222>  (144)..(144)
<223>  any amino acid residue


<220>
<221>  MISC_FEATURE
<222>  (377)..(377)
<223>  any amino acid residue


<220>
<221>  MISC_FEATURE
<222>  (395)..(395)
<223>  any amino acid residue


<400>  1

Met Trp Gln Phe Ile Arg Ser Arg Ile Leu Thr Val Ile Ile Phe Ile
1               5                   10                  15


Gly Ala Ala His Gly Leu Leu Val Val Gly Pro Lys Phe Ile Arg Ala
            20                  25                  30


Asn Gln Glu Tyr Thr Leu Val Ile Ser Asn Phe Asn Ser Gln Leu Ser
        35                  40                  45


Lys Val Asp Leu Leu Leu Lys Leu Glu Gly Glu Thr Asp Asn Gly Leu
    50                  55                  60


Ser Val Leu Asn Val Thr Lys Met Val Asp Val Arg Arg Asn Met Asn
65                  70                  75                  80


Arg Met Ile Asn Phe Asn Met Pro Glu Xaa Leu Thr Ala Gly Asn Tyr

                85                  90                  95

Lys Ile Thr Ile Asp Gly Gln Arg Gly Phe Ser Phe His Lys Glu Ala
            100                 105                 110

Glu Leu Val Tyr Leu Ser Lys Ser Ile Ser Gly Leu Ile Gln Val Asp
        115                 120                 125

Lys Pro Val Phe Lys Pro Gly Asp Thr Val Asn Phe Arg Val Ile Xaa
    130                 135                 140

Leu Asp Thr Glu Leu Lys Pro Pro Ala Arg Val Lys Ser Val Tyr Val
145                 150                 155                 160

Thr Ile Arg Asp Pro Gln Arg Asn Val Ile Arg Lys Trp Ser Thr Ala
                165                 170                 175

Lys Leu Tyr Ala Gly Val Phe Glu Ser Asp Leu Gln Ile Val Pro Thr
            180                 185                 190

Pro Met Leu Gly Val Trp Asn Ile Ser Val Glu Val Glu Gly Glu Glu
        195                 200                 205

Leu Val Ser Lys Thr Phe Glu Val Lys Glu Tyr Val Leu Ser Thr Phe
    210                 215                 220

Asp Val Gln Val Met Pro Ser Val Ile Pro Leu Glu Glu His Gln Ala
225                 230                 235                 240

Val Asn Leu Thr Ile Glu Ala Asn Tyr His Phe Gly Lys Pro Val Gln
                245                 250                 255

Gly Val Ala Lys Val Glu Leu Tyr Leu Asp Asp Asp Lys Leu Asn Gln
            260                 265                 270

Lys Lys Glu Leu Thr Val Tyr Gly Lys Gly Gln Val Glu Leu Arg Phe
        275                 280                 285

Asp Asn Phe Ala Met Asp Ala Asp Gln Gln Asp Val Arg Val Lys Val
    290                 295                 300

Ser Phe Ile Glu Gln Tyr Thr Asn Arg Thr Val Val Lys Gln Ser Gln
305                 310                 315                 320

Ile Thr Val Tyr Arg Tyr Ala Tyr Arg Val Glu Leu Ile Lys Glu Ser
                325                 330                 335

Pro Gln Phe Arg Pro Gly Leu Pro Phe Lys Cys Ala Leu Gln Phe Thr
            340                 345                 350

His His Asp Gly Thr Pro Ala Lys Gly Ile Thr Gly Lys Val Glu Val
        355                 360                 365

Ser Asp Val Gly Phe Glu Thr Thr Xaa Thr Ser Asp Asn Asp Gly Leu
    370                 375                 380

Ile Lys Leu Glu Leu Gln Pro Ser Glu Gly Xaa Glu Gln Leu Gly Ile
385                 390                 395                 400

Asn Phe Asn Ala Val Asp Gly Phe Phe Phe Tyr Glu Asp Val Asn Lys
                405                 410                 415

Val Glu Thr Val Thr Asp Ala Tyr Ile Lys Leu Glu Leu Lys Ser Pro
            420                 425                 430

Ile Lys Arg Asn Lys Leu Met Arg Phe Met Val Thr Cys Thr Glu Arg
        435                 440                 445

Met Thr Phe Phe Val Tyr Tyr Val Met Ser Lys Gly Asn Ile Ile Asp
    450                 455                 460

Ala Gly Phe Met Arg Pro Asn Lys Gln Thr Lys Tyr Leu Leu Gln Leu
465                 470                 475                 480

Asn Ala Thr Glu Lys Met Ile Pro Lys Ala Lys Ile Leu Ile Ala Thr
                485                 490                 495

Val Ala Gly Arg Thr Val Val Tyr Asp Tyr Ala Asp Leu Asp Phe Gln
            500                 505                 510

Glu Leu Arg Asn Asn Phe Asp Leu Ser Ile Asp Glu Gln Glu Ile Lys
        515                 520                 525

Pro Gly Arg Gln Ile Glu Leu Ser Met Ser Gly Arg Pro Gly Ala Tyr
    530                 535                 540

Val Gly Leu Ala Ala Tyr Asp Lys Ala Leu Leu Leu Phe Asn Lys Asn
545                 550                 555                 560

His Asp Leu Phe Trp Glu Asp Ile Gly Gln Val Phe Asp Gly Phe His
                565                 570                 575

Ala Ile Asn Glu Asn Glu Phe Asp Ile Phe His Ser Leu Gly Leu Phe
            580                 585                 590

Ala Arg Thr Leu Asp Asp Ile Leu Phe Asp Ser Ala Asn Glu Lys Thr
        595                 600                 605

Gly Arg Asn Ala Leu Gln Ser Gly Lys Pro Ile Gly Lys Leu Val Ser

610                 615                 620

Tyr Arg Thr Asn Phe Gln Glu Ser Trp Leu Trp Lys Asn Val Ser Ile
625                 630                 635                 640

Gly Arg Ser Gly Ser Arg Lys Leu Ile Glu Val Val Pro Asp Thr Thr
                645                 650                 655

Thr Ser Trp Tyr Leu Thr Gly Phe Ser Ile Asp Pro Val Tyr Gly Leu
            660                 665                 670

Gly Ile Ile Lys Lys Pro Ile Gln Phe Thr Thr Val Gln Pro Phe Tyr
        675                 680                 685

Ile Val Glu Asn Leu Pro Tyr Ser Ile Lys Arg Gly Glu Ala Val Val
    690                 695                 700

Leu Gln Phe Thr Leu Phe Asn Asn Leu Gly Ala Glu Tyr Ile Ala Asp
705                 710                 715                 720

Val Thr Leu Tyr Asn Val Ala Asn Gln Thr Glu Phe Val Gly Arg Pro
                725                 730                 735

Asp Thr Asp Leu Ser Tyr Thr Lys Ser Val Ser Val Pro Pro Lys Val
            740                 745                 750

Gly Val Pro Ile Ser Phe Leu Ile Lys Ala Arg Lys Leu Gly Glu Met
        755                 760                 765

Ala Val Arg Val Lys Ala Ser Ile Met Leu Gly His Glu Thr Asp Ala
    770                 775                 780

Leu Glu Lys Val Ile Arg Val Met Pro Glu Ser Leu Ala Gln Pro Lys
785                 790                 795                 800

Met Asp Thr Ser Phe Phe Cys Phe Asp Asp Tyr Lys Asn Gln Thr Phe
                805                 810                 815

Pro Phe Asn Leu Asp Ile Asn Lys Lys Ala Asp Asn Gly Ser Lys Lys
            820                 825                 830

Ile Glu Phe Arg Leu Asn Pro Asn Leu Leu Thr Met Val Ile Lys Asn
        835                 840                 845

Leu Asp Asn Leu Leu Ala Val Pro Thr Gly Cys Gly Glu Gln Asn Met
    850                 855                 860

Val Lys Phe Val Pro Asn Ile Leu Val Leu Asp Tyr Leu Tyr Ala Thr
865                 870                 875                 880

Gly Ser Lys Glu Gln His Leu Ile Asp Lys Ala Thr Asn Leu Leu Arg
                885                 890                 895

Gln Gly Tyr Gln Asn Gln Met Arg Tyr Arg Gln Thr Asp Gly Ser Phe
            900                 905                 910

Gly Val Trp Glu Lys Ser Gly Ser Ser Val Phe Leu Thr Ala Phe Val
        915                 920                 925

Ala Thr Ser Met Gln Thr Ala Ser Lys Tyr Met Asn Asp Ile Asp Ala
    930                 935                 940

Ala Met Val Glu Lys Ala Leu Asp Trp Leu Ala Ser Lys Gln His Ser
945                 950                 955                 960

Ser Gly Arg Phe Asp Glu Thr Gly Lys Val Trp His Lys Asp Met Gln
                965                 970                 975

Gly Gly Leu Arg Asn Gly Val Ala Leu Thr Ser Tyr Val Leu Thr Ala
            980                 985                 990

Leu Leu Glu Asn Asp Ile Ala Lys  Val Lys His Ala Val  Val Ile Gln
        995                 1000                1005

Asn Gly  Met Asn Tyr Leu Ser  Asn Gln Leu Ala Phe  Ile Asn Asn
    1010                 1015                 1020

Pro Tyr  Asp Leu Ser Ile Ala  Thr Tyr Ala Met Met  Leu Asn Gly
    1025                 1030                 1035

His Thr  Met Lys Lys Glu Ala  Leu Asp Lys Leu Ile  Asp Met Ser
    1040                 1045                 1050

Ile Ser  Asp Asn Asn Lys Lys  Glu Arg Tyr Trp Gly  Thr Thr Asn
    1055                 1060                 1065

Gln Ile  Glu Thr Thr Ala Tyr  Ala Leu Leu Ser Phe  Val Met Ala
    1070                 1075                 1080

Glu Lys  Tyr Leu Asp Gly Ile  Pro Val Met Asn Trp  Leu Val Asn
    1085                 1090                 1095

Gln Arg  Tyr Val Thr Gly Ser  Phe Pro Arg Thr Gln  Asp Thr Phe
    1100                 1105                 1110

Val Gly  Leu Lys Ala Leu Thr  Lys Leu Ala Glu Lys  Ile Ser Pro
    1115                 1120                 1125

Ser Arg  Asn Asp Tyr Thr Val  Gln Leu Lys Tyr Lys  Lys Asn Thr

1130                1135                1140

Lys Tyr Phe Asn Ile Asn Ser Glu Gln Ile Asp Val Gln Asn Phe
    1145                1150                1155

Leu Glu Ile Pro Glu Asp Thr Lys Lys Leu Glu Ile Asn Val Gly
    1160                1165                1170

Gly Ile Gly Phe Gly Leu Leu Glu Val Ile Tyr Gln Phe Asp Leu
    1175                1180                1185

Asn Leu Val Asn Phe Glu His Arg Phe Lys Leu Asp Leu Glu Lys
    1190                1195                1200

Gln Asn Thr Gly Ser Asp Tyr Glu Leu Arg Leu Arg Val Cys Ala
    1205                1210                1215

Asn Tyr Ile Pro Glu Leu Thr Asp Ser Gln Ser Asn Met Ala Leu
    1220                1225                1230

Ile Glu Val Thr Leu Pro Ser Gly Tyr Val Val Asp Arg Asn Pro
    1235                1240                1245

Ile Ser Glu Gln Thr Thr Val Asn Pro Ile Gln Asn Met Glu Ile
    1250                1255                1260

Arg Tyr Gly Gly Thr Ser Val Val Leu Tyr Tyr Tyr Lys Met Gly
    1265                1270                1275

Thr Glu Arg Asn Cys Phe Thr Val Thr Ala Tyr Arg Arg Phe Lys
    1280                1285                1290

Val Ala Leu Lys Arg Pro Ala Tyr Val Val Val Tyr Asp Tyr Tyr
    1295                1300                1305

Asn Thr Asn Leu Asn Ala Ile Lys Val Tyr Glu Val Asp Lys Gln
    1310                1315                1320

Asn Val Cys Glu Ile Cys Glu Glu Glu Asp Cys Pro Ala Glu Cys
    1325                1330                1335

Lys Lys
    1340

<210>  2
<211>  1158
<212>  PRT
<213>  Anopheles gambiae

<400>  2

Phe Glu Ser Asp Leu Gln Ile Ala Pro Thr Pro Met Leu Gly Val Trp
1               5                   10                  15

Asn Ile Ser Val Glu Val Glu Gly Glu Glu Leu Val Ser Lys Thr Phe
            20                  25                  30

Glu Val Lys Glu Tyr Val Leu Ser Thr Phe Asp Val Gln Val Met Pro
        35                  40                  45

Ser Val Ile Pro Leu Glu Glu His Gln Ala Val Asn Leu Thr Ile Glu
    50                  55                  60

Ala Asn Tyr His Phe Gly Lys Pro Val Gln Gly Val Ala Lys Val Glu
65                  70                  75                  80

Leu Tyr Leu Asp Asp Asp Lys Leu Lys Leu Lys Lys Glu Leu Thr Val
                85                  90                  95

Tyr Gly Lys Gly Gln Val Glu Leu Arg Phe Asp Asn Phe Ala Met Asp
            100                 105                 110

Ala Asp Gln Gln Asp Val Pro Val Lys Val Ser Phe Val Glu Gln Tyr
        115                 120                 125

Thr Asn Arg Thr Val Val Lys Gln Ser Gln Ile Thr Val Tyr Arg Tyr
    130                 135                 140

Ala Tyr Arg Val Glu Leu Ile Lys Glu Ser Pro Gln Phe Arg Pro Gly
145                 150                 155                 160

Leu Pro Phe Lys Cys Ala Leu Gln Phe Thr His His Asp Gly Thr Pro
                165                 170                 175

Ala Lys Gly Ile Ser Gly Lys Val Glu Val Ser Asp Val Arg Phe Glu
            180                 185                 190

Thr Thr Thr Thr Ser Asp Asn Asp Gly Leu Ile Lys Leu Glu Leu Gln
        195                 200                 205

Pro Ser Glu Gly Thr Glu Gln Leu Ser Ile His Phe Asn Ala Val Asp
    210                 215                 220

Gly Phe Phe Phe Tyr Glu Asp Val Asn Lys Val Glu Thr Val Thr Asp
225                 230                 235                 240

Ala Tyr Ile Lys Leu Glu Leu Lys Ser Pro Ile Lys Arg Asn Lys Leu
                245                 250                 255

Met Arg Phe Met Val Thr Cys Thr Glu Arg Met Thr Phe Phe Val Tyr
            260                 265                 270

Tyr Val Met Ser Lys Gly Asn Ile Ile Asp Ala Gly Phe Met Arg Pro
      275                 280                 285

Asn Lys Gln Pro Lys Tyr Leu Leu Gln Leu Asn Ala Thr Glu Lys Met
    290                 295                 300

Ile Pro Arg Ala Lys Ile Leu Ile Ala Thr Val Ala Gly Arg Thr Val
305                 310                 315                 320

Val Tyr Asp Phe Ala Asp Leu Asp Phe Gln Glu Leu Arg Asn Asn Phe
                325                 330                 335

Asp Leu Ser Ile Asp Glu Gln Glu Ile Lys Pro Gly Arg Gln Ile Glu
            340                 345                 350

Leu Ser Met Ser Gly Arg Pro Gly Ala Tyr Val Gly Leu Ala Ala Tyr
      355                 360                 365

Asp Lys Ala Leu Leu Leu Phe Asn Lys Asn His Asp Leu Phe Trp Glu
    370                 375                 380

Asp Ile Gly Gln Val Phe Asp Gly Phe His Ala Ile Asn Glu Asn Glu
385                 390                 395                 400

Phe Asp Ile Phe His Ser Leu Gly Leu Phe Ala Arg Thr Leu Asp Asp
                405                 410                 415

Ile Leu Phe Asp Ser Ala Asn Glu Lys Thr Gly Arg Asn Ala Leu Gln
            420                 425                 430

Ser Gly Lys Pro Ile Gly Lys Leu Val Ser Tyr Arg Thr Asn Phe Gln
      435                 440                 445

Glu Ser Trp Leu Trp Lys Asn Val Ser Ile Gly Arg Ser Gly Ser Arg
    450                 455                 460

Lys Leu Ile Glu Val Val Pro Asp Thr Thr Thr Ser Trp Tyr Leu Thr
465                 470                 475                 480

Gly Phe Ser Ile Asp Pro Val Tyr Gly Leu Gly Ile Ile Lys Lys Pro
                485                 490                 495

Ile Gln Phe Thr Thr Val Gln Pro Phe Tyr Ile Val Glu Asn Leu Pro
            500                 505                 510

Tyr Ser Ile Lys Arg Gly Glu Ala Val Val Leu Gln Phe Thr Leu Phe
      515                 520                 525

Asn Asn Leu Gly Ala Glu Tyr Ile Ala Asp Val Thr Leu Tyr Asn Val
    530                 535                 540

Ala Asn Gln Thr Glu Phe Val Gly Arg Pro Asn Thr Asp Leu Ser Tyr
545                 550                 555                 560

Thr Lys Ser Val Ser Val Pro Pro Lys Val Gly Val Pro Ile Ser Phe
                565                 570                 575

Leu Ile Lys Ala Arg Lys Leu Gly Glu Met Ala Val Arg Val Lys Ala
            580                 585                 590

Ser Ile Met Leu Gly His Glu Thr Asp Ala Leu Glu Lys Val Ile Arg
        595                 600                 605

Val Met Pro Glu Ser Leu Val Gln Pro Arg Met Asp Thr Arg Phe Phe
    610                 615                 620

Cys Phe Asp Asp His Lys Asn Gln Thr Phe Pro Ile Asn Leu Asp Ile
625                 630                 635                 640

Asn Lys Lys Ala Asp Ser Gly Ser Thr Lys Ile Glu Phe Arg Leu Asn
                645                 650                 655

Pro Asn Leu Leu Thr Thr Val Ile Lys Asn Leu Asp His Leu Leu Gly
            660                 665                 670

Val Pro Thr Gly Cys Gly Glu Gln Asn Met Val Lys Phe Val Pro Asn
        675                 680                 685

Ile Leu Val Leu Asp Tyr Leu His Ala Ile Gly Ser Lys Glu Gln His
    690                 695                 700

Leu Ile Asp Lys Ala Thr Asn Leu Leu Arg Gln Gly Tyr Gln Asn Gln
705                 710                 715                 720

Met Arg Tyr Arg Gln Thr Asp Gly Ser Phe Gly Leu Trp Glu Thr Thr
                725                 730                 735

Asn Gly Ser Val Phe Leu Thr Ala Phe Val Gly Thr Ser Met Gln Thr
            740                 745                 750

Ala Val Lys Tyr Ile Ser Asp Ile Asp Ala Ala Met Val Glu Lys Ala
        755                 760                 765

Leu Asp Trp Leu Ala Ser Lys Gln His Phe Ser Gly Arg Phe Asp Lys
    770                 775                 780

Ala Gly Ala Glu Tyr His Lys Glu Met Gln Gly Gly Leu Arg Asn Gly
785                 790                 795                 800

Val Ala Leu Thr Ser Tyr Val Leu Met Ala Leu Leu Glu Asn Asp Ile
                805                 810                 815

Ala Lys Ala Lys His Ala Glu Val Ile Gln Lys Gly Met Thr Tyr Leu
            820                 825                 830

Ser Asn Gln Phe Gly Ser Ile Asn Asn Ala Tyr Asp Leu Ser Ile Ala
        835                 840                 845

Thr Tyr Ala Met Met Leu Asn Gly His Thr Met Lys Glu Glu Ala Leu
    850                 855                 860

Asn Lys Leu Ile Asp Met Ser Phe Ile Asp Ala Asp Lys Asn Glu Arg
865                 870                 875                 880

Phe Trp Asn Thr Thr Asn Pro Ile Glu Thr Thr Ala Tyr Ala Leu Leu
                885                 890                 895

Ser Phe Val Met Ala Glu Lys Tyr Thr Asp Gly Ile Pro Val Met Asn
            900                 905                 910

Trp Leu Val Asn Gln Arg Tyr Val Thr Gly Ser Phe Pro Ser Thr Gln
        915                 920                 925

Asp Thr Phe Val Gly Leu Lys Ala Leu Thr Lys Met Ala Glu Lys Ile
    930                 935                 940

Ser Pro Ser Arg Asn Asp Tyr Thr Val Gln Leu Lys Tyr Lys Lys Ser
945                 950                 955                 960

Ala Lys Tyr Phe Lys Ile Asn Ser Glu Gln Ile Asp Val Glu Asn Phe
                965                 970                 975

Val Asp Ile Pro Glu Asp Thr Lys Lys Leu Glu Ile Asn Val Gly Gly
            980                 985                 990

Ile Gly Phe Gly Leu Leu Glu Val Val Tyr Gln Phe Asn Leu Asn Leu
        995                 1000                1005

Val Asn Phe Glu Asn Arg Phe Gln Leu Asp Leu Glu Lys Gln Asn
    1010                1015                1020

Thr Gly Ser Asp Tyr Glu Leu Arg Leu Lys Val Cys Ala Ser Tyr
    1025                1030                1035

Ile Pro Gln Leu Thr Asp Arg Arg Ser Asn Met Ala Leu Ile Glu
    1040                1045                1050

Val Thr Leu Pro Ser Gly Tyr Val Val Asp Arg Asn Pro Ile Ser
    1055                1060                1065

Glu Gln Thr Lys Val Asn Pro Ile Gln Lys Thr Glu Ile Arg Tyr
    1070                1075                1080

Gly Gly Thr Ser Val Val Leu Tyr Tyr Asp Asn Met Gly Ser Glu
    1085                1090                1095

Arg Asn Cys Phe Thr Leu Thr Ala Tyr Arg Arg Phe Lys Val Ala
    1100                1105                1110

Leu Lys Arg Pro Ala Tyr Val Val Val Tyr Asp Tyr Tyr Asn Thr
    1115                1120                1125

Asn Leu Asn Ala Ile Lys Val Tyr Glu Val Asp Lys Gln Asn Leu
    1130                1135                1140

Cys Glu Ile Cys Asp Glu Glu Asp Cys Pro Ala Glu Cys Lys Lys
    1145                1150                1155

<210>  3
<211>  1331
<212>  PRT
<213>  Anopheles gambiae

<220>
<221>  MISC_FEATURE
<222>  (1207)..(1207)
<223>  any amino acid residue

<400>  3

Met Pro Trp Tyr Val Arg Met Leu Leu Val Ile Ser Leu Leu Gly Ser
1               5                   10                  15

Ser Trp Gly Val Leu Val Val Gly Pro Lys Phe Val Arg Ser Asn Gln
            20                  25                  30

Glu Tyr Ala Leu Val Ile Ser Asn Phe Asn Ser Gly Ser Ser Lys Val
        35                  40                  45

Asn Leu Met Leu His Met Glu Gly Phe Ser Lys Asn Gln Thr Ser Val
    50                  55                  60

Phe Ala Ile Arg Lys Pro Val Asp Val Arg Arg Phe Met Ser Arg Ile
65                  70                  75                  80

Val Ser Phe Asp Ile Pro Asn Ile Ala Ser Pro Val Asp Ile Lys Leu
                85                  90                  95

Thr Met Val Gly Gln Arg Gly Phe Ser Phe His Glu Glu Glu His Leu

            100                 105                 110

Val His Arg Ser Lys Ser Ile Ser Gly Leu Ile Gln Ile Asp Lys Pro
        115                 120                 125

Val Phe Arg Pro Gly Asp Leu Val Lys Phe Arg Ala Ile Val Leu Asp
    130                 135                 140

Thr Glu Leu Lys Pro Pro Ala Arg Ile Lys Ser Val Asn Val Thr Ile
145                 150                 155                 160

Gln Asp Pro His Gln Asn Lys Ile Arg Gly Trp Pro Val Ala Lys Leu
                165                 170                 175

Tyr Ala Gly Val Phe Glu Asn Asp Leu Gln Leu Ala Pro Ala Pro Leu
            180                 185                 190

Leu Gly Val Trp Asn Ile Thr Val Gln Val Gly Glu Glu Gln Leu Val
        195                 200                 205

Phe Lys Thr Phe Glu Val Lys Glu Tyr Val Leu Thr Ser Tyr Asp Val
    210                 215                 220

Gln Val Met Pro Ser Val Ile Pro Leu Val Glu His Gln Thr Leu Asn
225                 230                 235                 240

Leu Thr Ile Val Ala Asn Tyr His Phe Gly Lys Pro Val Gln Gly Val
                245                 250                 255

Ala Lys Val Glu Leu Tyr Leu Val Asp Asp Thr Leu Asp Gln Lys Lys
            260                 265                 270

Glu Leu Thr Met Tyr Gly Met Gly Gln Val Glu Leu Arg Phe Asn Glu
        275                 280                 285

Leu Leu Glu Leu Tyr Glu Asp Gln Gln Asp Val Arg Val Lys Phe Thr
    290                 295                 300

Phe Thr Glu Gln His Thr Asn Arg Thr Val Val Lys Glu Gln Ala Ile
305                 310                 315                 320

Thr Val Tyr Lys His Pro Tyr Arg Ala Gln Leu Thr Lys Glu Ser Pro
                325                 330                 335

Gln Phe Arg Pro Gly Thr Pro Phe Lys Cys Thr Leu Thr Leu Ile Tyr
            340                 345                 350

His Asp Gly Arg Pro Ala Gly His Val Pro Phe Phe Val Asn Val Glu
        355                 360                 365

Gly Glu Asp Val Asp His Gln Gln Thr Tyr Thr Thr Gly His Asp Gly
    370                 375                 380

Thr Ile Lys Leu Leu Met Arg Pro Thr Glu Leu Thr Glu Thr Ile Asp
385                 390                 395                 400

Ile Thr Val Ser Glu Asp Asn Ser Glu Phe Thr Tyr Thr Glu Arg Ile
                405                 410                 415

Glu Lys Val His Ala Asp Thr Asn Val Phe Leu Lys Leu Glu Leu Lys
            420                 425                 430

Ser Pro Ile Lys Leu Gly Lys Leu Ile Arg Leu Met Val Thr Cys Asn
        435                 440                 445

Glu Arg Met Thr Phe Phe Ile Tyr Tyr Val Ile Ser Lys Gly Asn Ile
    450                 455                 460

Val Asp Ala Gly Phe Val Arg Pro Asn Arg Gln Thr Lys Phe Met Phe
465                 470                 475                 480

Gln Leu Thr Ala Ser Glu Lys Met Ile Pro Lys Ala His Ile Phe Val
                485                 490                 495

Ala Thr Val Ser Gln Asp Val Val Val Trp Asp Ser Leu Glu Ile Asp
            500                 505                 510

Leu Lys Gln Phe Ser Asn His Leu Asp Ile Ile Ile Asp Glu Lys Glu
        515                 520                 525

Leu Lys Pro Gly Gln Glu Ile Glu Leu Leu Leu Lys Gly Arg Pro Ser
    530                 535                 540

Ala Tyr Val Gly Leu Ala Ala Tyr Asp Lys Gly Leu Leu Ala Tyr Ser
545                 550                 555                 560

Lys Gln His Asp Leu Phe Trp Glu Asp Val Met Gln Val Phe Asp Thr
                565                 570                 575

Phe His Ala Thr Asp Gln Asn Glu Phe Asp Val Phe Asn Ser Met Gly
            580                 585                 590

Leu Phe Ala Arg Leu Ser Gly Gly Asn Arg Ile Gly Ala Ser Pro Thr
        595                 600                 605

Thr Thr Glu Arg Phe Gly Ser Ala Ala Ser Arg Pro Ile Ser Arg Leu
    610                 615                 620

Val Ala Tyr Arg Thr Asn Phe Leu Glu Ser Trp Leu Trp Gln Asn Val

625                 630                 635                 640

Ser Ile Gly Arg Thr Gly Ser Arg Thr Val His Glu Val Leu Pro Asp
                645                 650                 655

Thr Thr Thr Ser Trp Tyr Leu Thr Gly Phe Ser Ile Asp Pro Val Tyr
            660                 665                 670

Gly Leu Gly Ile Ile Lys Lys Pro Ile Glu Phe Ile Thr Val Lys Pro
        675                 680                 685

Phe Tyr Ile Val Asp Ser Leu Pro Tyr Ser Ile Lys Arg Gly Glu Ala
    690                 695                 700

Ala Val Leu Gln Phe Thr Leu Phe Asn Asn Leu Gly Ala Glu Tyr Ile
705                 710                 715                 720

Ala Asp Val Thr Leu Tyr Asn Val Ala Asn Gln Thr Glu Phe Ile Glu
                725                 730                 735

Arg Pro Asp Lys Asp Leu Ser Tyr Thr Lys Ser Val Ser Val Pro Pro
            740                 745                 750

Lys Val Gly Val Pro Ile Ser Phe Gly Val Lys Ala Arg Lys Leu Gly
        755                 760                 765

Glu Met Val Val Arg Ile Lys Ala Ser Ile Met Thr Gly Lys Glu Thr
    770                 775                 780

Asp Ala Met Glu Lys Val Ile Arg Val Ile Pro Glu Asn Ile Met Phe
785                 790                 795                 800

Glu Lys Thr Glu Thr Arg Phe Phe Ser Met Asp Glu Tyr Gly Lys Gln
                805                 810                 815

Glu Phe Asn Met Gln Leu Asp Ile Pro Lys Asn Ile Ser Thr Val Gln
            820                 825                 830

Ile Glu Cys Arg Ile Ser Ser Asn Leu Leu Ser Pro Val Ile His Asn
        835                 840                 845

Leu Asp Ser Leu Leu Asp Val Pro Ser Ala Ser Gly Ala Pro Ser Met
    850                 855                 860

Ile Asn Phe Ile Pro Pro Leu Val Val Leu Asp Tyr Leu Lys Ala Val
865                 870                 875                 880

Ser Ser Thr Thr Thr His Leu Ile Glu Lys Ala Thr Gly Leu Leu Arg
                885                 890                 895

Asn Gly Tyr Gln Leu Glu Leu Lys Tyr Arg Gln Arg Asp Gly Ser Phe
            900                 905                 910

Gly Asn Trp Arg Asp Ser Lys Gly Ser Val Phe Val Thr Ala Leu Val
        915                 920                 925

Gly Thr Ser Leu Glu Ala Ala Ser Lys His Ile Thr Glu Val Asp Leu
    930                 935                 940

Thr Leu Val Asp Arg Leu Phe Glu Trp Leu Ala Ala Lys Gln His Ser
945                 950                 955                 960

Ser Gly Arg Phe Asp Glu Glu Gln Pro Ile Thr Tyr His Ser Leu Gln
                965                 970                 975

Gly Gly Ser Arg Asn Gly Ile Ala Leu Thr Ser Phe Val Leu Ile Ala
            980                 985                 990

Phe Leu Gln Asn Thr Lys Ala Ser Ala Gln His Arg Ser Ile Ile Glu
        995                 1000                1005

Lys Gly Ile Gln Tyr Val Ala Asn Gln Leu Glu Ser Ile Ala Asp
    1010                1015                1020

Val Tyr Asp Leu Ser Leu Ala Thr Tyr Ala Leu Met Leu Ala Asp
    1025                1030                1035

His Arg Gln Lys Ser Ser Ala Leu Asn Lys Leu Ile Glu Leu Gly
    1040                1045                1050

Ile Ala Thr Asn Glu Thr Arg Tyr Trp Pro Arg His Thr Ala Ser
    1055                1060                1065

Ile Glu Thr Thr Ala Tyr Ala Leu Leu Ser Leu Val His Ala Lys
    1070                1075                1080

Arg Tyr Ala Asp Gly Leu Met Val Met His Trp Leu Val Asn Gln
    1085                1090                1095

Gln Ser Ala Thr Gly Ser Phe Pro Arg Thr Gln Asp Thr Phe Val
    1100                1105                1110

Gly Ile Arg Ala Leu Ala Ala Leu Ser Glu Ala Ile Ala Pro Gln
    1115                1120                1125

Lys Asn Asp Tyr Thr Ala Ile Val Leu His Gly Lys Ala Arg Lys
    1130                1135                1140

Val Tyr Lys Val Ala Ala Ser Glu Ala Asp Gln Glu Tyr Arg Asp

1145                1150                1155

Glu Leu Pro Gly Asp Ser Lys Leu Val Arg Phe Ser Ala Asn Gly
    1160                1165                1170

Arg Gly Leu Gly Met Phe Thr Val Ala Phe Gln Tyr Gly Ile Asp
    1175                1180                1185

Val Arg Asn Ile Glu His Gly Phe Ser Leu Arg Leu Val Asp Gln
    1190                1195                1200

Phe Ser Asn Xaa Ala Tyr Thr Leu Gln Leu Gln Val Cys Thr Ser
    1205                1210                1215

Phe Ser Pro Gln Leu Met His Thr Arg Ser Asn Leu Ala Leu Val
    1220                1225                1230

Glu Val Asn Phe Pro Ser Gly Tyr Val Val Ser Arg Lys Ser Leu
    1235                1240                1245

Val Asp Glu Thr Arg Arg Asn Pro Phe Lys Asp Val Glu Val Arg
    1250                1255                1260

Tyr Gly Gln Thr Ser Leu Val Ile Tyr Tyr Glu Thr Leu Gly Pro
    1265                1270                1275

Glu Glu Asn Cys Phe Ser Val Thr Ala Asn Arg Leu Phe Arg Val
    1280                1285                1290

Ala Phe His Arg Gln Ala Tyr Val Met Val His Asp Thr Tyr Asp
    1295                1300                1305

Glu Lys Phe Arg Ala Ile Lys Phe Tyr Gln Val Pro His Asp Gly
    1310                1315                1320

Ala Asp Ile Gln Ser Tyr Leu Asp
    1325                1330

<210>  4
<211>  1239
<212>  PRT
<213>  Anopheles gambiae

<400>  4

Ser Trp Asn Arg Phe Ile Ser Glu Arg Val Gln Ala Lys Met Arg His
1               5                   10                  15

Leu Ala Ser Ala Ser Leu Leu Val Ile Thr Leu Leu Ile Asn Phe Val
            20                  25                  30

His Gly Ile Leu Ile Val Gly Pro Lys His Ile Arg Ala Gly Gln Asn
        35                  40                  45

Tyr Thr Val Ile Leu Ser Ser Phe Glu Ser Asn Thr Asn Glu Arg Asn
    50                  55                  60

Leu Leu Leu Gln Leu Asp Gly Leu Ser Ala Gly Gly Lys Lys Leu Leu
65                  70                  75                  80

His Leu Gly Gln Pro Thr Arg Val Gln Pro Leu Ser Asn Ser Val Ile
                85                  90                  95

Ser Phe Lys Leu Pro Glu Ser Leu Pro Ala Gly Ser Tyr Lys Leu Thr
            100                 105                 110

Ile Asp Gly Gln Asn Gly Phe Lys Phe His Arg Glu Ala Asp Leu Leu
        115                 120                 125

Leu Ala Ala Ser Thr Thr Thr Ala Leu Val Gln Leu Ser Lys Pro Ile
    130                 135                 140

Tyr Lys Pro Gly Asp Val Leu Gln Phe Arg Val Ile Val Leu Gly Gly
145                 150                 155                 160

Asp Leu Lys Pro Pro Ala Pro Ser Val Thr Ala Thr Val Ile Val His
                165                 170                 175

Asp Pro Gln Arg Asn Val Ile Arg Arg Trp Thr Ala Val Ser Leu Gln
            180                 185                 190

Leu Gly Val Phe Glu Glu Gln Leu Gln Ile Gly Thr Val Pro Leu Leu
        195                 200                 205

Gly Arg Tyr Thr Ile Thr Val Thr Val Thr Gly Ala Asn Glu Ile Val
    210                 215                 220

Ser Lys Thr Phe Asp Val Arg Glu Tyr Val Leu Pro Ala Phe Glu Val
225                 230                 235                 240

Ala Val Lys Ala Arg Ala Val Pro Leu Glu Lys His Gln Arg Leu Asn
                245                 250                 255

Leu Thr Leu Ser Ala Arg Tyr Tyr Thr Gly Gln Pro Val Arg Gly Val
            260                 265                 270

Ala Thr Val Glu Leu Tyr Leu Glu Asp Asp Lys Leu Asp Gln Arg Arg
        275                 280                 285

Val Val Gly Val Tyr Gly Ala Ile Gln Leu Asp Leu Pro Phe Asn Glu
    290                 295                 300

His Leu Ser Val Tyr Asp Asp Ser Gln Asp Val Arg Val His Val Ala
305                 310                 315                 320

Phe Thr Gln Asp Glu Thr Ser Lys Leu Thr Phe Val Arg Glu Leu Gln
                325                 330                 335

Ile Val Phe Phe Ser Val Lys Ile Gly Lys Pro Phe Lys Leu Lys Val
            340                 345                 350

Thr Cys Asn Glu Glu Leu Ser Phe Phe Met Tyr Tyr Val Val Ser Arg
        355                 360                 365

Gly Val Ile Val Asp Ser Ala Phe Leu Gln Pro Lys Lys Val Thr Glu
    370                 375                 380

His Phe Ile Glu Ile Glu Ala Ser Asp Gln Met Val Pro Arg Ser Lys
385                 390                 395                 400

Val Ile Val Val Thr Val Ala Lys Asn Val Val Leu Cys Asp Phe Val
                405                 410                 415

Asp Ile Asp Phe Glu Asp Leu Arg Asn Asn Ser Gln Phe Asp Leu Gln
            420                 425                 430

Ile Asp Arg Thr Glu Ile Arg Pro Gly Asp Gln Leu Gln Leu Asn Met
        435                 440                 445

Arg Gly Pro Pro Gly Ala Phe Val Ala Leu Ala Ala Tyr Asp Lys Ser
    450                 455                 460

Leu Leu Gln Tyr Ser Asn Asn His Asp Ile Phe Trp Glu Asp Val Trp
465                 470                 475                 480

Gly Val Phe Asp Lys Phe Tyr Ser Val Glu Arg Asn Glu Phe Asp Leu
                485                 490                 495

Phe His Ser Leu Gly Leu Phe Ala Arg Thr Met Glu His Ile Thr Phe
            500                 505                 510

Asp Lys Ala Asn Asp Gln Thr Ala Arg Asp Gly Ser Ser Ser Ser Lys
        515                 520                 525

Asn Gly Pro Ser Asn Ser Gln Ala Ser Phe Arg Thr Asn Phe Leu Glu
    530                 535                 540

Ser Trp Leu Trp Lys Thr Asp Lys Ile Gly Ser Ser Gly Ser Ala Thr
545                 550                 555                 560

Thr Lys Glu Ser Val Pro Asp Thr Ile Thr Ala Trp His Leu Thr Gly
                565                 570                 575

Phe Ser Ile Asp Pro Val Tyr Gly Leu Gly Ile Ile Lys Gln Pro Leu
            580                 585                 590

Gln Leu Thr Thr Val Gln Pro Phe Tyr Ile Val Pro Asn Met Pro Tyr
        595                 600                 605

Ser Ile Lys Arg Gly Glu Leu Val Glu Leu Gln Phe Ile Val Phe Asn
    610                 615                 620

Asn Phe Pro Lys Lys Tyr Lys Ala Ser Val Thr Leu Phe Ser Val Asp
625                 630                 635                 640

Asn Gln Thr Glu Phe Val Gly Arg Pro Ser Thr Glu Thr Ser Tyr Thr
                645                 650                 655

Lys Ser Ile Glu Ala Ser Pro Asp Thr Gly Val Pro Val Ala Phe Leu
            660                 665                 670

Ile Lys Ala Arg Lys Leu Gly Glu Met Thr Val Arg Val Asp Ala Ser
        675                 680                 685

Ile Glu Pro Ala Lys Asp Ser Ile Glu Ser Val Ile Arg Val Ile Pro
    690                 695                 700

Glu Ser Leu Val Lys Arg Glu Met Ile Ser Arg Phe Phe Cys His Asn
705                 710                 715                 720

Thr Tyr Gln Asn Gln Ser Phe Val Leu Gly Leu Asp Phe Asp Arg Lys
                725                 730                 735

Ala Asp Ala Gly Thr Arg Lys Ile Asp Phe Ile Leu Thr Arg Lys Leu
            740                 745                 750

Asn Ile Leu Thr Ser Val Met Asp Asn Leu Glu Ser Leu Leu Ser Val
        755                 760                 765

Pro Thr Gly Cys Gly Glu Gln Asn Met Met Arg Leu Val Pro Ile Val
    770                 775                 780

Leu Val Leu Asp Tyr Leu Thr Ser Ile Gly Ser Ala Asp Lys Gln Leu
785                 790                 795                 800

Thr Ala Lys Ala Ile Gly Leu Leu Arg Ala Gly Tyr Gln Asn Gln Met
                805                 810                 815

Arg Tyr Arg Gln Pro Asp Gly Ser Phe Gly Leu Trp Glu Lys Ser Gly
            820                 825                 830

Gly Ala Val Phe Leu Thr Ala Phe Val Gly Lys Thr Leu Ala Thr Ala
        835                 840                 845

Ala Lys Tyr Ile Ser Glu Ile Glu Pro Ser Met Val Glu Gln Ala Phe
    850                 855                 860

Asp Trp Leu Ala Ala Arg Gln His Ser Thr Gly Arg Phe Asp Glu Val
865                 870                 875                 880

Gly Pro Val Phe His Arg Asp Met Gln Gly Gly Leu Arg Gln Gly Ile
                885                 890                 895

Ala Leu Thr Ser Phe Val Leu Ile Ala Leu Leu Glu Gln Pro Lys Val
            900                 905                 910

Ala Thr Lys His Arg Ala Ala Ile Glu Lys Gly Ile Asp Tyr Val Thr
        915                 920                 925

Gln Thr Leu Gly Ser Ile Glu Asp Ser Tyr Asp Leu Ala Ile Ala Thr
    930                 935                 940

Tyr Ala Leu Leu Leu Gln Lys His Ser Ser Gly Glu Arg Phe Leu Glu
945                 950                 955                 960

Lys Leu Ile Gly Leu Ser Thr Val Gln Gln Asn Gly Thr Glu Arg Phe
                965                 970                 975

Trp Ala Arg Asp Ala His Gly Ile Glu Thr Thr Ala Tyr Gly Leu Leu
            980                 985                 990

Ser Phe Val Leu Ala Glu Lys Tyr Val Asp Gly Thr Ser Ile Met Arg
        995                 1000                1005

Trp Leu Val Lys Gln Arg Tyr Thr Pro Gly Ser Phe Pro Arg Thr
    1010                1015                1020

Gln Asp Thr Phe Val Gly Leu Lys Ala Leu Thr Lys Leu Ala Glu
    1025                1030                1035

Lys Ile Ser Pro Ser Arg Asn Asp Tyr Ser Val Gln Leu Arg His
    1040                1045                1050

Ala Gly Arg Lys Lys Glu Phe Arg Val Thr Ser Gln Asp Ile Gly
    1055                1060                1065

Thr Leu Gln Asn Ala Gln Gln Gly Val Asp Glu Thr Ala Gln Leu
    1070                1075                1080

Glu Leu His Val Ala Gly Ile Gly Phe Gly Leu Leu Gln Val Val
    1085                1090                1095

Tyr Glu Tyr Gly Val Asp Leu Arg Asn Phe Thr Ala Gln Phe Val
    1100                1105                1110

Leu Glu Leu Gln Lys Ser Val Thr Asn Ala Asn His Gln Leu Gln
    1115                1120                1125

Leu Glu Val Cys Ser Ser Phe Thr Pro Gln Leu Ser Asp Gly Arg
    1130                1135                1140

Ser Asn Met Val Leu Val Glu Val Asn Phe Pro Ser Gly Tyr Thr
    1145                1150                1155

Val Glu Gln Arg Gly Gln Pro Ile Thr Gly Ala Thr Lys His Asn
    1160                1165                1170

Pro Ile Gln Gln Lys Thr Glu Val Arg Phe Gly Ala Thr Ser Val
    1175                1180                1185

Val Val Tyr Tyr Asn Ser Met Gly Pro Glu Arg Asn Cys Phe Thr
    1190                1195                1200

Ile Thr Ala Tyr Arg Arg Gln Lys Val Thr Leu Lys Arg Pro Ala
    1205                1210                1215

Tyr Val Leu Val His Asp Tyr Tyr Ser Glu Leu Asn Ala Ile Lys
    1220                1225                1230

Met Tyr Gln Val Asp Asp
    1235

<210>  5
<211>  880
<212>  PRT
<213>  Anopheles gambiae

<220>
<221>  MISC_FEATURE
<222>  (726)..(727)
<223>  any amino acid residues

<400>  5

Met Trp Lys Cys Ile Arg Ser Arg Ile Leu Met Val Ile Ile Val Ile
1               5                   10                  15

Gly Ala Ala Gln Gly Val Leu Val Val Gly Pro Lys Tyr Ile Arg Ala
            20                  25                  30

Asn Gln Glu Tyr Thr Leu Val Ile Ser Asn Ile Asn Ser Asn Gln Thr

35                  40                  45

Lys Leu Asp Leu Ile Val Lys Leu Glu Gly Glu Thr Asp Asn Gly Leu
    50                  55                  60

Asn Val Leu Asn Val Thr Lys Thr Phe Asp Val Arg Arg Asn Met Asn
65                  70                  75                  80

Arg Met Ile Asn Phe Asn Met Pro Glu Asn Leu Ala Glu Gly Asn Tyr
                85                  90                  95

Lys Ile Thr Ile Asp Gly Gln Gln Gly Phe Ser Phe His Lys Glu Ala
            100                 105                 110

Ala Leu Val Tyr Leu Ser Lys Ser Ile Ser Gly Leu Ile Gln Val Asp
        115                 120                 125

Lys Pro Val Phe Lys Pro Gly Asp Thr Val Asn Phe Arg Val Ile Val
    130                 135                 140

Leu Asp Thr Glu Leu Lys Pro Pro Ala Arg Val Lys Leu Val Tyr Val
145                 150                 155                 160

Thr Ile Gln Asp Pro Gln Arg Asn Val Ile Arg Lys Trp Ser Ser Ala
                165                 170                 175

Lys Leu Tyr Thr Gly Val Phe Glu Gly Tyr Leu Gln Ile Ala Pro Thr
            180                 185                 190

Ala Met Leu Gly Ile Trp Asn Ile Leu Val Gln Val Glu Gly Glu Glu
        195                 200                 205

Leu Val Ser Lys Thr Phe Glu Val Lys Glu Tyr Val Leu Ser Thr Phe
    210                 215                 220

Asp Val Gln Val Met Pro Ser Val Ile Pro Leu Glu Glu His Gln Ala
225                 230                 235                 240

Val Asn Leu Thr Ile Glu Ala Tyr Tyr His Phe Gly Lys Pro Val Gln
                245                 250                 255

Gly Val Ala Lys Val Glu Leu Tyr Leu Asp Asp Glu Leu Ile Asp Gln
            260                 265                 270

Lys Lys Glu Leu Thr Val Tyr Gly Lys Gly Gln Val Glu Leu Leu Leu
        275                 280                 285

Phe Gly Lys Phe Glu Met Tyr Glu Asp Gln Gln Asp Val Arg Val Lys
    290                 295                 300

Val Thr Phe Ile Glu His Tyr Thr Asn Arg Thr Leu Val Lys Leu Ser
305                 310                 315                 320

Gln Ile Thr Ile Tyr Lys His Ala Tyr Arg Val Glu Leu Ile Lys Glu
                325                 330                 335

Ser Pro His Phe His Pro Gly Ile Pro Phe Lys Phe Val Leu Gln Phe
            340                 345                 350

Thr His His Asp Gly Thr Pro Ala Lys Gly Ile Thr Gly Lys Val Glu
        355                 360                 365

Val Ser Gly Ile Gly Phe Glu Thr Thr Ala Thr Ser Asp Asn Asp Gly
    370                 375                 380

Leu Ile Lys Leu Glu Leu Gln Lys Asn Glu Asp Ile Glu Leu Met His
385                 390                 395                 400

Val Ser Phe Val Asn Asn Asn Asp Gly Phe Val Phe Glu His Asn Val
                405                 410                 415

Asp Arg Asp Val Phe Ala Thr Asn Ala Tyr Ile Lys Ile Glu Leu Lys
            420                 425                 430

Ser Pro Ile Lys Leu Asn Lys Asn Met Arg Phe Thr Val Thr Cys Thr
        435                 440                 445

Glu Arg Met Thr Phe Phe Val Tyr Tyr Val Val Ser Lys Gly Asn Ile
    450                 455                 460

Val Asp Ala Gly Phe Met Arg Pro Asn Lys Gln Lys Lys Tyr Ser Leu
465                 470                 475                 480

Gln Leu Asp Ala Thr Glu Lys Met Ile Pro Lys Ala Lys Thr Leu Val
                485                 490                 495

Ala Thr Leu Val Asn Arg Thr Met Val Asn Asp Ile Val Asp Ile Asp
            500                 505                 510

Phe Gln Gly Phe Arg Asn Asn Phe Asp Leu Ser Ile Asp Glu Gln Glu
        515                 520                 525

Ile Lys Pro Gly Arg Gln Thr Glu Leu Ser Met Ser Gly Arg Pro Gly
    530                 535                 540

Ala Tyr Val Gly Leu Ala Ala Tyr Asp Lys Ala Leu Leu Leu Phe Asn
545                 550                 555                 560

Lys Asn His Asp Leu Phe Trp Glu Asp Ile Gly Gln Val Phe Asp Gly

                565                 570                 575

Phe His Ser Tyr Asp Thr Asn Glu Phe Asp Leu Phe His Asn Met Gly
            580                 585                 590

Leu Phe Ala Arg Met Asp Asn Ile Met Phe Asp Gln Ser Asn Asp Lys
        595                 600                 605

Ser Ala Arg Ser Gly Gln Gln Ala Asp Gly Thr Val Phe Arg Lys Gln
    610                 615                 620

Phe Leu Glu Ser Trp Leu Trp Lys Thr Ala Ile Ile Gly Asn Ser Gly
625                 630                 635                 640

Thr Leu Lys Leu Ile Glu Val Val Pro Asp Thr Thr Thr Thr Trp Tyr
                645                 650                 655

Leu Thr Gly Phe Ser Ile Asp Pro Val Tyr Pro Asn Leu Gln Glu Glu
            660                 665                 670

Tyr Thr Val Asp Val Thr Leu Tyr Asn Glu Asn Asn Glu Met Glu Phe
        675                 680                 685

Leu Gly Arg Ser Ile Ser Asn Val Ser Tyr Thr Lys Ser Val Ser Val
    690                 695                 700

Pro Pro Lys Val Gly Lys Pro Val Ser Phe Leu Val Lys Ala Lys Lys
705                 710                 715                 720

Leu Gly Glu Met Met Xaa Xaa Leu Asn Leu Val Asn Phe Glu His Arg
                725                 730                 735

Phe Gln Leu Asp Leu Gln Lys Gln Asn Thr Ser Ser Asp Asn Glu Leu
            740                 745                 750

Arg Leu Lys Val Cys Ala Asn Tyr Ile Pro Thr Leu Arg Asp Ser His
        755                 760                 765

Ser Asn Met Thr Leu Ile Glu Val Thr Leu Pro Ser Gly Tyr Val Val
    770                 775                 780

Asp Arg Asn Pro Ile Ser Glu Gln Thr Thr Val Asn Pro Ile Gln Asn
785                 790                 795                 800

Met Glu Ile Arg Tyr Gly Gly Thr Ser Val Val Leu Tyr Tyr Tyr Asn
                805                 810                 815

Met Gly Thr Glu Arg Asn Cys Phe Thr Val Thr Ala Tyr Arg Arg Phe
            820                 825                 830

Lys Val Ala Leu Lys Arg Pro Ala Tyr Val Val Val Tyr Asp Tyr Tyr
        835                 840                 845

Asp Thr Asn Gln Asn Ala Ile Lys Val Tyr Glu Met Asp Lys Gln Asn
    850                 855                 860

Val Cys Glu Ile Cys Asp Ala Cys Asp Cys Ile Ala Glu Cys Lys Thr
865                 870                 875                 880

<210> 6
<211> 1314
<212> PRT
<213> Anopheles gambiae

<400> 6

Met Ser Lys Ser Ile Met Trp Lys Cys Ile Arg Ser Arg Ile Leu Met
1               5                   10                  15

Val Ile Ile Val Ile Gly Ala Ala His Gly Leu Leu Val Val Gly Pro
            20                  25                  30

Lys Phe Ile Arg Ala Asn Gln Glu Tyr Thr Leu Val Ile Ser Asn Phe
        35                  40                  45

Asn Ser Gln Leu Ser Lys Val Asp Leu Leu Leu Lys Leu Glu Gly Glu
    50                  55                  60

Thr Asp Asn Gly Leu Ser Val Leu Asn Val Thr Lys Met Val Asp Val
65                  70                  75                  80

Arg Arg Asn Met Asn Arg Met Ile Asn Phe Asn Met Pro Glu Asp Leu
                85                  90                  95

Thr Ala Gly Asn Tyr Lys Ile Thr Ile Asp Gly Gln Arg Gly Phe Ser
            100                 105                 110

Phe His Lys Glu Ala Glu Leu Val Tyr Leu Ser Lys Ser Ile Ser Gly
        115                 120                 125

Leu Ile Gln Val Asp Lys Pro Val Phe Lys Pro Gly Asp Thr Val Asn
    130                 135                 140

Phe Arg Val Ile Val Leu Asp Thr Glu Leu Lys Pro Pro Ala Arg Val
145                 150                 155                 160

Lys Ser Val Tyr Val Thr Ile Arg Asp Pro Gln Arg Asn Val Ile Arg
                165                 170                 175

Lys Trp Ser Thr Ala Lys Leu Tyr Ala Gly Val Phe Glu Ser Asp Leu
            180                 185                 190

Gln Ile Ala Pro Thr Pro Met Leu Gly Val Trp Asn Ile Ser Val Glu
        195                 200                 205

Val Glu Gly Glu Glu Leu Val Ser Lys Thr Phe Glu Val Lys Glu Tyr
    210                 215                 220

Val Leu Ser Thr Phe Asp Val Gln Val Met Pro Ser Val Ile Pro Leu
225                 230                 235                 240

Glu Glu His Gln Ala Val Asn Leu Thr Ile Glu Ala Asn Tyr His Phe
                245                 250                 255

Gly Lys Pro Val Gln Gly Val Ala Lys Val Glu Leu Tyr Leu Asp Asp
            260                 265                 270

Asp Lys Leu Lys Leu Lys Lys Glu Leu Thr Val Tyr Gly Lys Gly Gln
        275                 280                 285

Val Glu Leu Arg Phe Asp Asn Phe Ala Met Asp Ala Asp Gln Gln Asp
    290                 295                 300

Val Pro Val Lys Val Ser Phe Val Glu Gln Tyr Thr Asn Arg Thr Val
305                 310                 315                 320

Val Lys Gln Ser Gln Ile Thr Val Tyr Arg Tyr Ala Tyr Arg Val Glu
                325                 330                 335

Leu Ile Lys Glu Ser Pro Gln Phe Arg Pro Gly Leu Pro Phe Lys Cys
            340                 345                 350

Ala Leu Gln Phe Thr His His Asp Gly Thr Pro Ala Lys Gly Ile Ser
        355                 360                 365

Gly Lys Val Glu Val Ser Asp Val Arg Phe Glu Thr Thr Thr Thr Ser
    370                 375                 380

Asp Asn Asp Gly Leu Ile Lys Leu Glu Leu Gln Pro Ser Glu Gly Thr
385                 390                 395                 400

Glu Gln Leu Ser Ile His Phe Asn Ala Val Asp Gly Phe Phe Phe Tyr
                405                 410                 415

Glu Asp Val Asn Lys Val Glu Thr Val Thr Asp Ala Tyr Ile Lys Leu
            420                 425                 430

Glu Leu Lys Ser Pro Ile Lys Arg Asn Lys Leu Met Arg Phe Met Val
        435                 440                 445

Thr Cys Thr Glu Arg Met Thr Phe Phe Val Tyr Tyr Val Met Ser Lys
    450                 455                 460

Gly Asn Ile Ile Asp Ala Gly Phe Met Arg Pro Asn Lys Gln Thr Lys
465                 470                 475                 480

Tyr Leu Leu Gln Leu Asn Ala Thr Glu Lys Met Ile Pro Lys Ala Lys
                485                 490                 495

Ile Leu Val Ala Thr Leu Val Asn Arg Thr Met Val Asn Asp Ile Val
            500                 505                 510

Asp Ile Asp Phe Gln Gly Phe Arg Ser Asn Phe Asp Leu Ser Ile Asp
        515                 520                 525

Glu Gln Glu Ile Lys Pro Gly Arg Gln Ile Glu Leu Ser Met Ser Gly
    530                 535                 540

Arg Pro Gly Ala Tyr Val Gly Leu Ala Ala Tyr Asp Lys Ala Leu Leu
545                 550                 555                 560

Leu Phe Asn Lys Asn His Asp Leu Phe Trp Glu Asp Ile Gly Gln Asn
                565                 570                 575

Met Gly Leu Phe Ala Arg Met Asp Asn Ile Met Phe Asp Gln Ser Asn
            580                 585                 590

Asp Lys Ser Ala Arg Ser Gly Gln Gln Ala Asp Gly Thr Val Phe Arg
        595                 600                 605

Lys Gln Phe Leu Glu Ser Trp Leu Trp Lys Thr Ala Ile Ile Gly Asn
    610                 615                 620

Ser Gly Thr Leu Lys Leu Ile Glu Val Val Pro Asp Thr Thr Thr Thr
625                 630                 635                 640

Trp Tyr Leu Thr Gly Phe Ser Ile Asp Pro Val Tyr Gly Leu Gly Ile
                645                 650                 655

Val Lys Lys Pro Ile Glu Ile Thr Thr Val Gln Pro Phe Val Val Met
            660                 665                 670

Glu Ser Leu Pro Tyr Ser Ile Lys Arg Gly Glu Ala Ile Glu Ile Gln
        675                 680                 685

Phe Ile Leu Ile Ser Asn Leu Gln Glu Glu Tyr Thr Val Asp Val Thr
    690                 695                 700

Leu Tyr Asn Glu Asn Asn Glu Met Glu Phe Leu Gly Arg Ser Ile Ser
705                 710                 715                 720

Asn Val Ser Tyr Thr Lys Ser Val Ser Val Pro Pro Lys Val Gly Lys
                725                 730                 735

Pro Val Ser Phe Leu Val Lys Ala Lys Lys Leu Gly Glu Met Met Val
            740                 745                 750

Arg Val Lys Ala Ser Ile Ala Asn Glu Leu Ala Thr Asp Ala Leu Glu
        755                 760                 765

Lys Val Ile Arg Val Thr Pro Glu Ser Leu Val Gln Ser Gly Val Glu
    770                 775                 780

Ser Phe Gly Phe Phe Met Asp Thr His Gln Asn Arg Thr Phe Leu Val
785                 790                 795                 800

Asn Pro Asn Ile Asp Lys Lys Ala Asp Asn Gly Ser Ile Ala Ile Glu
                805                 810                 815

Leu Arg Val Asn Pro Asn Leu Leu Ile Lys Val Lys Glu Asn Leu Asn
            820                 825                 830

Asp Leu Arg Thr Val Ser Pro Thr Cys Lys Asn Val Ile Arg Leu Ala
        835                 840                 845

Leu Asn Phe Ala Val Ala Asp Tyr Leu Ile Ala Cys Gly Pro Lys Glu
    850                 855                 860

Gln Asn Leu Pro Glu Asn Ala Val Asp Ala Leu Ser Lys Glu Tyr Met
865                 870                 875                 880

Leu Leu Met Thr Cys Leu Asn Ser Asp Ser Ser Phe Asp Ser Val Gln
                885                 890                 895

Asn Ile Val Ser Asn Ile Phe Tyr Thr Ala Phe Val Ala Asn Thr Leu
            900                 905                 910

Asp Thr Ala Pro Lys Asn Val Cys Gln Thr Ser Asn Val Lys Leu Glu
        915                 920                 925

Lys Ala Phe Asp Trp Leu Ala Ser Arg Gln Gln Arg Phe Gly Ser Phe
    930                 935                 940

Arg Glu Ile Glu Thr Asp Leu His Tyr Thr Arg Ser Glu Ile Ala Leu
945                 950                 955                 960

Thr Ser Tyr Val Leu Ala Ser Met Leu Glu Asn Glu Ser Ala Lys Val
                965                 970                 975

Lys His Ala Val Val Ile Glu Lys Gly Met Trp Phe Leu Ser Asn Gln
            980                 985                 990

Phe Asp Leu Ile Thr Ser Ala Asn Asp Leu Ala Ile Val Thr Tyr Ala
        995                 1000                1005

Met Met Leu Tyr Gly His Trp Arg Lys Asp Ala Ala Phe Glu Lys
    1010                1015                1020

Leu Ile Asp Met Ser Thr Ile Thr Asn Asn Gly Ala Glu Arg Tyr
    1025                1030                1035

Trp Lys Thr Ser Asn Ser Val Glu Ala Thr Ser Tyr Ala Leu Leu
    1040                1045                1050

Ser His Val Leu Ser Asn Lys Leu Leu Ala Ala Leu Pro Ile Met
    1055                1060                1065

Arg Trp Leu Val Asn Gln Lys Ser Glu Ile Asp Ser Val Ser Gly
    1070                1075                1080

Gln Glu Thr Thr Tyr Leu Arg Leu Lys Ala Leu Ser Arg Met Thr
    1085                1090                1095

Asn Lys Ile Ser Pro Ser Arg Asn Asp Tyr Met Val Gln Leu Lys
    1100                1105                1110

Tyr Lys Gln Ser Thr Arg Leu Leu Arg Phe Asp Ser Tyr Arg Asn
    1115                1120                1125

Met Ile Gln Asn Ile Thr Thr Pro Gln Asp Leu Arg Lys Ile Lys
    1130                1135                1140

Ile Thr Val Glu Gly Ile Gly Val Gly Leu Leu Glu Val Met Tyr
    1145                1150                1155

Lys Tyr Arg Leu Asn Leu Val Asn Phe Glu His Arg Phe Gln Leu
    1160                1165                1170

Asp Leu Gln Lys Gln Asn Thr Ser Ser Asp Asn Glu Leu Arg Leu
    1175                1180                1185

Lys Val Cys Ala Asn Tyr Ile Pro Thr Leu Arg Asp Ser His Ser
    1190                1195                1200

Asn Met Thr Leu Ile Glu Val Thr Leu Pro Ser Gly Tyr Val Val
    1205                1210                1215

Asp Arg Asn Pro Ile Ser Glu Gln Thr Thr Val Asn Pro Ile Gln
    1220                1225                1230

Asn Met Glu Ile Arg Tyr Gly Gly Thr Ser Val Val Leu Tyr Tyr
    1235                1240                1245

Tyr Asn Met Gly Thr Glu Arg Asn Cys Phe Thr Val Thr Ala Tyr
    1250                1255                1260

Arg Arg Phe Lys Val Ala Leu Lys Arg Pro Ala Tyr Val Val Val
    1265                1270                1275

Tyr Asp Tyr Tyr Asp Thr Asn Gln Asn Ala Ile Lys Val Tyr Glu
    1280                1285                1290

Met Asp Lys Gln Asn Val Cys Glu Ile Cys Asp Ala Cys Gly Cys
    1295                1300                1305

Thr Ala Glu Cys Lys Thr
    1310

<210>  7
<211>  1347
<212>  PRT
<213>  Anopheles gambiae

<400>  7

Met Trp Gln Phe Ile Arg Ser Arg Ile Leu Thr Val Ile Ile Phe Ile
1               5                   10                  15

Ala Ala Ala His Gly Leu Leu Val Met Gly Pro Lys Cys Ile Arg Asp
            20                  25                  30

Asn Gln Asn Tyr Thr Leu Thr Ile Ser Asn Phe Tyr Ser Asn Pro Ser
        35                  40                  45

Lys Leu Asp Leu Met Val Lys Leu Glu Gly Lys Asn Asp Asn Gly Leu
    50                  55                  60

Ser Val Leu Asn Val Thr Lys Met Ile Asp Val Pro Cys Lys Ser Ile
65                  70                  75                  80

Arg Met Ile Asn Phe Ser Ile Leu Asp Ser Leu Ser Ser Gly Asn Tyr
                85                  90                  95

Lys Ile Thr Ile Asp Gly Leu Gln Gly Phe Asn Phe His Glu Glu Ala
            100                 105                 110

Glu Leu Val Tyr Leu Ser Lys Ser Ile Ser Gly Val Ile Gln Ile Asp
        115                 120                 125

Lys Pro Val Ile Lys Pro Gly Asp Thr Val Ile Phe Arg Val Ile Val

130                 135                 140

Leu Asp Thr Ala Leu Lys Pro Pro Val Arg Val Lys Phe Val Asn Val
145                 150                 155                 160

Ile Ile Arg Asp Pro Asn Asp Asn Val Ile Arg Arg Trp Pro Ala Ala
                165                 170                 175

Glu Leu Tyr Ala Gly Val Phe Glu Ser Asn Leu Gln Ile Ala Ile Thr
            180                 185                 190

Ser Leu Leu Gly Val Trp Asn Ile Ser Val Leu Val Glu Gly Ala Glu
        195                 200                 205

Ile Ala Ser Lys Thr Phe Glu Val Lys Lys Asp Val Phe Ser Trp Phe
    210                 215                 220

Asp Val Tyr Ile Lys Pro Ser Val Ile Pro Leu Glu Glu His Gln Ala
225                 230                 235                 240

Leu Asn Leu Thr Ile Glu Ala Lys Tyr Pro Phe Gly Lys Pro Val Gln
                245                 250                 255

Gly Val Ala Lys Val Glu Leu Tyr Leu Glu Gly Asp Arg Leu Asp Leu
            260                 265                 270

Ser Lys Asp Ile Asn Val Asn Gly Ser Glu Gln Val Met Leu Ala Phe
        275                 280                 285

Asn Gly Gly Leu Glu Leu Asn Asn Glu Gln Gln Asp Val Arg Val Lys
    290                 295                 300

Val Val Val Val Glu Gln Asp Thr Asp Arg Thr Val Met Lys Glu Gln
305                 310                 315                 320

Ser Ile Ser Val His Lys Tyr Pro Tyr Arg Leu Thr Ile Val Lys Asp
                325                 330                 335

Thr Leu Gln Phe Arg Pro Gly His His Phe Asn Cys Thr Val Leu Leu
            340                 345                 350

Thr Tyr His Asp Val Ser Pro Ala Arg Asn Val Ser Leu Leu Val Glu
        355                 360                 365

Ala Thr Gly Glu Gly Val Glu His Glu Gln Thr Tyr Phe Thr Gln His
    370                 375                 380

Asp Gly Ser Ile Asn Val Leu Leu His Pro Thr Leu Ser Thr Gln Glu
385                 390                 395                 400

Val Tyr Leu Thr Ile Ser Glu Lys Lys Tyr Asp Leu Leu Tyr Met Lys
                405                 410                 415

Lys Leu His Thr Val Gln Pro Leu Thr Asn Ile Thr Met Lys Leu Gln
            420                 425                 430

Leu Arg Phe Pro Ile Lys Gln Asn Glu Pro Ile Met Leu Leu Ala Thr
        435                 440                 445

Cys Ser Glu Arg Met Thr Phe Leu Ala Tyr Tyr Val Val Ser Lys Ser
    450                 455                 460

Asn Ile Val Asp Ala Gly Tyr Ile Gly Gln Leu Arg Gln Lys Lys Ile
465                 470                 475                 480

Val Phe Glu Ile Ser Ala Thr Glu Lys Met Leu Pro Lys Ala Thr Ile
                485                 490                 495

Tyr Val Thr Ser Ala Phe Glu Asn Ile Leu Ile Trp Asp Ala Leu Glu
            500                 505                 510

Ile Asp Leu Arg Pro Leu Ser Asn Asp Leu Glu Ile Arg Val Asn Lys
        515                 520                 525

Leu Lys Ala Lys Pro Gly Gln Glu Ile Glu Leu Glu Leu Lys Gly Arg
    530                 535                 540

Pro Ser Ala Tyr Val Gly Leu Ala Ala Tyr Asp Lys Gly Leu Leu Ala
545                 550                 555                 560

Phe Ala Asn His Leu Asp Pro Phe Trp Glu Asp Val Thr Gln Leu Phe
                565                 570                 575

Asp Tyr Ser Tyr Glu Asp Asp Gln Thr Glu Leu Asp Gly Phe His Gly
            580                 585                 590

Glu Gly Val Phe Leu Lys Ile Leu Gly Gly Phe Gln Leu Gly Asn Thr
        595                 600                 605

Leu Lys Val Thr Lys Arg Thr Val Lys Asp Val Ala Pro Pro Leu Leu
    610                 615                 620

Tyr His Ala Ser Leu Gln Arg Asn Cys Val Lys Ser Trp Leu Trp Arg
625                 630                 635                 640

Asn Val Thr Val Gly Ser Ser Gly Thr Leu Lys Leu Ala Glu Val Phe
                645                 650                 655

Pro Glu Ser Ile Thr Ser Trp Thr Leu Thr Gly Phe Ser Ile Asp Pro

660                 665                 670

Ser Tyr Gly Leu Ser Ile Ile Arg Lys Pro Ile Glu Leu Thr Thr Ile
        675                 680                 685

Gln Pro Phe Leu Ile Val Asp Tyr Leu Pro Tyr Ser Ile Lys Arg Glu
    690                 695                 700

Lys Glu Val Leu Leu Gln Ile Ala Leu Phe Asn Asn Phe Glu Gln Asp
705                 710                 715                 720

His Thr Val Asp Val Thr Leu Tyr Asn Met Lys Asn Glu Met Glu Ile
                725                 730                 735

Leu Gly Arg Pro Val Thr Asn Gln Ser Tyr Thr Lys Thr Val Ser Val
            740                 745                 750

Pro Pro Lys Val Leu Phe Pro Val Ser Phe Leu Val Lys Ala Gln Lys
        755                 760                 765

Leu Gly Glu Met Ala Val His Val Thr Ala Ser Ile Ala Asn Gly Leu
    770                 775                 780

Glu Thr Asp Ala Leu Glu Lys Val Ile Arg Val Met Pro Glu Ser Leu
785                 790                 795                 800

Val Gln Pro Arg Met Asp Arg Arg Phe Phe Cys Phe Asp Glu Tyr Lys
                805                 810                 815

Asn Gln Thr Phe Leu Ile Tyr Leu Asp Ile Ile Lys Lys Ala Asp Asn
            820                 825                 830

Gly Ser Ile Lys Ile Glu Phe Arg Leu Asn Pro Asn Leu Leu Thr Met
        835                 840                 845

Val Ile Lys Asn Leu Asp His Leu Leu Ala Leu Ser Ala Gly Thr Gly
    850                 855                 860

Glu Asp Asn Met Val Lys Phe Val Pro Asn Ile Leu Val Leu Asp Tyr
865                 870                 875                 880

Leu His Ala Ile Gly Ser Lys Glu Gln His Leu Ile Asp Lys Ala Met
                885                 890                 895

Asn Leu Val Arg Leu Gly Tyr Gln Asn Gln Met Arg Tyr Cys Gln Lys
            900                 905                 910

Asp Gly Ser Phe Gly Leu Trp Asp Asn Thr Gly Gly Asn Thr Phe Leu
        915                 920                 925

Thr Thr Phe Val Ala Thr Thr Met Gln Thr Ala Ser Lys Tyr Ile Val
    930                 935                 940

Glu Ile Asp Ala Glu Ile Val Asp Arg Ala Leu Asp Trp Leu Ala Ser
945                 950                 955                 960

Lys Gln His Ser Ser Gly Arg Ile Asp Glu Val Gly Ala Val Phe His
                965                 970                 975

Lys Asp Met Lys Gly Gly Leu Arg Asp Gly Val Ala Leu Thr Ser Phe
            980                 985                 990

Val Leu Thr Ala Leu Leu Glu Asn  Asn Ile Ala Lys Val  Lys Tyr Ala
        995                 1000                 1005

Glu Ala  Ile Gln Lys Gly Met  Ser Tyr Leu Ser Asn  Gln Phe Gly
    1010                 1015                 1020

Ser Ile  Asn Asn Ala Tyr Asp  Leu Ser Ile Ala Thr  Tyr Ala Met
    1025                 1030                 1035

Met Leu  Asn Gly His Ile Met  Lys Glu Glu Ser Leu  Asn Lys Leu
    1040                 1045                 1050

Ile Asn  Met Ser Phe Ile Asn  Ala Asp Lys Asn Glu  Arg Phe Trp
    1055                 1060                 1065

Asn Thr  Thr Asn Pro Ile Glu  Thr Thr Ala Tyr Ala  Leu Leu Ser
    1070                 1075                 1080

Phe Val  Met Ala Glu Lys Tyr  Leu Asp Gly Ile Pro  Val Met Asn
    1085                 1090                 1095

Trp Leu  Val Asn Gln Arg Tyr  Val Thr Gly Ser Phe  Pro Ser Thr
    1100                 1105                 1110

Gln Asp  Thr Phe Val Gly Leu  Lys Ala Leu Thr Lys  Met Ala Glu
    1115                 1120                 1125

Lys Ile  Ser Pro Ser Arg Asn  Asp Tyr Thr Val Gln  Leu Lys Tyr
    1130                 1135                 1140

Gly Lys  Asp Ser Arg Ile Phe  Arg Ile Asn Ser Glu  His Ile Asp
    1145                 1150                 1155

Val Met  Asn Tyr Val Gly Ile  Pro Glu Asp Thr Arg  Lys Ile Asp
    1160                 1165                 1170

Ile Asn  Val Arg Gly Ile Gly  Phe Gly Leu Leu Glu  Val Ile Tyr

1175                1180                1185

Gln Tyr  Asp Leu Asn Leu Val  Lys Phe Glu Asn Arg  Phe Lys Leu
    1190                1195                1200

Asp Leu  Glu Lys Gln Asn Ile  Gly Ser Asp Lys Met  Ile Leu Asn
    1205                1210                1215

Val Cys  Ala Ser Phe Ile Pro  Met Tyr Leu Gln Ser  Gln Ser Asn
    1220                1225                1230

Met Ala  Leu Ile Glu Val Thr  Leu Pro Ser Gly Tyr  Val Val Asp
    1235                1240                1245

Arg Asn  Pro Ile Ser Glu Gln  Thr Thr Val Asn Pro  Ile Lys Lys
    1250                1255                1260

Ile Glu  Thr Arg Tyr Gly Gly  Thr Ser Val Val Val  Tyr Tyr Asp
    1265                1270                1275

Asn Met  Asp Asp Lys Lys His  Cys Phe Thr Val Ser  Ala His Arg
    1280                1285                1290

Gln Gln  Met Pro Thr Leu Arg  Arg Pro Ala Tyr Val  Val Val Tyr
    1295                1300                1305

Asp Tyr  Tyr Asp Thr Asn Ile  Asn Ala Ile Lys Met  Tyr Gln Leu
    1310                1315                1320

Glu Asp  Leu Ser Ala Cys Glu  Ile Ser Gly Glu Val  Asp Cys Ser
    1325                1330                1335

Glu Val  Cys Lys Thr Lys Gly  Gln Val
    1340                1345

<210>  8
<211>  119
<212>  PRT
<213>  Anopheles gambiae

<400>  8

Met Arg Gln Phe Ile Arg Ser Arg Ile Leu Thr Val Ile Ile Phe Ile
1               5                   10                  15

Ala Ala Ala His Gly Leu Leu Val Met Gly Pro Lys Cys Ile Arg Asp
            20                  25                  30

Asn Gln Asn Tyr Thr Leu Thr Ile Ser Asn Phe Tyr Ser Asn Pro Ser
        35                  40                  45

Lys Leu Asp Leu Met Val Lys Leu Glu Gly Lys Asn Asp Asn Gly Leu
    50                  55                  60

Ser Val Leu Asn Val Thr Lys Met Val Asp Val Pro Cys Lys Ser Ile
65                  70                  75                  80

Arg Ile Ile Asp Phe Ser Ile Pro Asp Ser Leu Ser Ser Gly Asn Tyr
                85                  90                  95

Lys Ile Thr Ile Asp Gly Leu Gln Gly Phe Asn Phe His Glu Glu Ala
            100                 105                 110

Glu Leu Val Tyr Leu Ser Lys
        115

<210>  9
<211>  948
<212>  PRT
<213>  Anopheles gambiae

<400>  9

Ser Lys Arg Val Leu Ser Phe Ile Arg Asp Ser Arg Ser Ser Val His
1               5                   10                  15

Phe Ser Leu His Thr Met Met Glu Arg Trp Leu Lys Ala Phe Pro Phe
            20                  25                  30

Asp Ala Val Asp Ala Phe Leu Phe Tyr Glu Asp Val Asn Lys Val Glu
        35                  40                  45

Thr Asp Ala Phe Ile Lys Leu Glu Leu Lys Ser Pro Ile Lys Leu Asn
    50                  55                  60

Lys Leu Met His Phe Thr Val Thr Cys Thr Glu Arg Met Ser Phe Phe
65                  70                  75                  80

Val Tyr Tyr Val Val Ser Lys Ser Asn Ile Ile Asp Ala Gly Phe Met
                85                  90                  95

Leu Ala Asn Lys Glu Thr Thr Phe Leu Leu Gln Leu Asn Ala Thr Glu
            100                 105                 110

Asn Met Ile Pro Lys Ala Lys Ile Leu Ile Ile Thr Val Ala Gly Arg
        115                 120                 125

Thr Val Met Tyr Asp Tyr Ile Asn Leu Asp Phe Gln Glu Leu Arg Asn
    130                 135                 140

Asn Phe Asp Leu Ser Val Asp Glu Gln Glu Ile Lys Pro Gly Arg Gln
145                 150                 155                 160

Ile Glu Leu Ser Met Ser Gly Arg Pro Gly Ala Tyr Val Gly Leu Ala
                165                 170                 175

Ala Tyr Asp Lys Ala Leu Leu Leu Phe Asn Lys Asn His Asp Leu Phe
            180                 185                 190

Trp Glu Asp Phe Leu Lys Val Phe Asp Gly Phe His Ser Tyr Asp Thr
        195                 200                 205

Asn Glu Phe Asp Leu Phe His Thr Met Gly Leu Phe Ala Arg Thr Leu
    210                 215                 220

Asp Asp Phe Phe Val Gln Asn Tyr Asn His Lys Ser Glu Arg Asn Gly
225                 230                 235                 240

Gln Gln Met Glu Gln Thr Val Val Arg Lys Gln Phe Val Glu Ser Trp
                245                 250                 255

Leu Trp Lys Asn Val Thr Ile Gly Ser Ser Gly Ser Leu Lys Leu Thr
            260                 265                 270

Glu Val Val Pro Asp Thr Thr Thr Ser Trp Tyr Leu Thr Gly Phe Ser
        275                 280                 285

Ile Asp Pro Val Val Gly Leu Gly Ile Ile Lys Lys Pro Ile Glu Phe
    290                 295                 300

Thr Thr Val Gln Pro Phe Phe Ile Leu Glu Ser Leu Pro Tyr Ser Ile
305                 310                 315                 320

Lys Arg Gly Glu Ala Ile Glu Ile Gln Phe Ile Leu Ile Ser Asn Leu
                325                 330                 335

Gln Gly Glu His Thr Val Asp Val Thr Leu Tyr Asn Glu Asn Asn Glu
            340                 345                 350

Met Glu Phe Ile Gly Arg Ser Ile Ala Asn Ala Ser Tyr Thr Lys Ser
        355                 360                 365

Val Ser Val Leu Pro Lys Trp Gly Lys Pro Ile Ser Phe Leu Val Lys
    370                 375                 380

Ala Lys Lys Leu Gly Glu Met Met Val Arg Val Lys Ala Ser Ile Ala
385                 390                 395                 400

Asn Gly Leu Ala Thr Asp Ala Leu Glu Lys Val Ile Arg Val Thr Pro
                405                 410                 415

Glu Ser Leu Glu Gln Ser Arg Val Glu Ser Phe Gly Ile Phe Phe Asp

            420                 425                 430

Glu Tyr Gln Asn Gln Thr Phe Ser Val Phe Pro Asn Ile Pro Lys Asn
        435                 440                 445

Ala Asp Asn Gly Ser Val Glu Ile Glu Leu Arg Val Asn Pro Asn Leu
    450                 455                 460

Leu Ile Thr Val Lys Glu Asn Leu Asn Asp Ile Arg Thr Asp Trp Ser
465                 470                 475                 480

Glu Cys Ser Lys Gly Ile Arg Phe Thr Leu Asn Phe Ala Val His Asn
                485                 490                 495

Tyr Leu Val Ala Ile Gly Ser Ser Asp Gln Ile Ser Ser Asp Asp Asn
            500                 505                 510

Ala Thr Leu Ser Lys His Tyr Ile Arg Leu Glu Lys Cys Phe Asn Ser
        515                 520                 525

Asn Gly Ser Trp Arg Asn Lys Val Phe Asp Thr Ala Phe Leu Val Asn
    530                 535                 540

Ala Leu His Asn Ala Met Lys Tyr Val Ser Trp Lys Asp Lys Arg Lys
545                 550                 555                 560

Leu Glu Lys Ala Phe Asp Trp Leu Ala Ser Gln Gln His His Ser Gly
                565                 570                 575

Ser Phe Lys Glu Thr Glu Ser Asp Leu His Tyr Lys Arg Ser Asp Val
            580                 585                 590

Ala Leu Thr Ser Tyr Val Leu Ala Val Met Leu Glu Asn Glu Ser Ala
        595                 600                 605

Lys Val Glu His Ala Val Val Ile Glu Lys Gly Met Ser Phe Leu Ser
    610                 615                 620

Asp Gln Leu Glu Ser Ile Thr Ser Ala Asn Asp Leu Ala Ile Val Thr
625                 630                 635                 640

Tyr Ala Met Met Ser Tyr Gly His Arg Leu Arg Asp Ala Ala Phe Glu
                645                 650                 655

Lys Leu Ile Tyr Met Ser Thr Ile Thr Asn Asn Gly Ala Glu Arg Tyr
            660                 665                 670

Trp Asn Pro Ser Asn Ser Val Glu Ala Thr Ser Phe Ala Leu Leu Ser
        675                 680                 685

His Val Val Ala Asn Lys Leu Gln Gly Ala Leu Pro Ile Met Arg Trp
    690                 695                 700

Leu Val Asn His Arg Ser Glu Leu Asn Ser Val Ser Gly Gln Gln Asn
705                 710                 715                 720

Thr Tyr Leu Arg Phe Lys Ala Leu Ser Ser Ile Ala Lys Lys Ile Ala
                725                 730                 735

Leu Ser Arg Asn Asp Tyr Asp Val Arg Ile Lys Tyr Arg Leu Ser Arg
            740                 745                 750

Lys Leu Phe Arg Phe Thr Ser Tyr Asp Ser Asp Thr Gln His Met Leu
        755                 760                 765

Ile Thr Gln Asn Val Arg Lys Leu Glu Ile His Val Gly Gly Ile Gly
    770                 775                 780

Ala Gly Leu Leu Gln Val Phe Tyr Arg Tyr Asn Leu Asn Leu Met Asn
785                 790                 795                 800

Phe Glu His Arg Phe Lys Leu Asp Leu Gln Lys Gln Ile Thr Asn Phe
                805                 810                 815

Asp Gln Glu Leu Lys Leu Asn Val Cys Ala Asn Tyr Ile Pro Thr Val
            820                 825                 830

Ser Glu Ser Arg Ser Asn Met Ala Leu Val Glu Val Thr Leu Pro Ser
        835                 840                 845

Gly Tyr Val Val Asp His Asn Pro Ile Ser Glu Gln Thr Thr Val Asn
    850                 855                 860

Pro Ile Gln Arg Ile Glu Ile Arg Tyr Gly Gly Thr Ser Val Val Leu
865                 870                 875                 880

Tyr Tyr Tyr Ser Met Gly Ser Glu Arg Asn Cys Phe Thr Val Ser Ala
                885                 890                 895

Tyr Arg Arg Phe Lys Val Ala Leu Lys Arg Pro Ala Tyr Val Val Val
            900                 905                 910

Tyr Asp Tyr Tyr Asp Thr Asn Leu Asn Ala Ile Lys Val Tyr Glu Val
        915                 920                 925

Asp Lys Gln Asn Val Cys Glu Ile Cys Glu Glu Glu Asp Cys Arg Ala
    930                 935                 940

Glu Cys Lys Ile

945

<210>  10
<211>  957
<212>  PRT
<213>  Anopheles gambiae

<400>  10

Ser Lys Arg Val Leu Ser Phe Ile Arg Asp Ser Arg Ser Ser Val His
1               5                   10                  15

Phe Ser Leu His Thr Met Met Glu Arg Trp Leu Lys Ala Phe Pro His
            20                  25                  30

Leu Glu Gly Ser Ser Leu Leu Gln Phe Asp Ala Val Asp Ala Phe Leu
        35                  40                  45

Phe Tyr Glu Asp Val Asn Lys Val Glu Thr Asp Ala Phe Ile Lys Leu
    50                  55                  60

Glu Leu Lys Ser Pro Ile Lys Leu Asn Lys Leu Met Arg Phe Ile Val
65                  70                  75                  80

Thr Cys Thr Glu Arg Met Thr Phe Phe Val Tyr Tyr Val Val Ser Lys
                85                  90                  95

Ser Asn Ile Val Asp Ala Gly Phe Val Arg Pro Lys Asn Glu Thr Thr
            100                 105                 110

Phe Leu Leu Gln Leu Tyr Ala Thr Glu Lys Met Phe Pro Lys Ala Lys
        115                 120                 125

Met Leu Val Ala Thr Val Thr Gly Arg Thr Val Val Tyr Asp Tyr Met
    130                 135                 140

Tyr Leu Asp Phe Gln Val Phe His Asn Asn Phe Thr Leu Ser Val Asp
145                 150                 155                 160

Glu Gln Glu Ile Lys Pro Arg Arg Gln Ile Glu Leu Ser Met Ser Gly
                165                 170                 175

Arg Pro Gly Ala Tyr Val Gly Leu Ala Ala Tyr Asp Lys Ala Leu Leu
            180                 185                 190

Leu Phe Asn Lys Asn His Asp Leu Phe Trp Glu Asp Phe Leu Lys Val
        195                 200                 205

Phe Asp Gly Phe His Ser Tyr Asp Thr Asn Glu Phe Asp Leu Phe His
    210                 215                 220

Thr Met Gly Leu Phe Ala Arg Thr Leu Asp Asp Phe Phe Val Gln Asn
225                 230                 235                 240

Tyr Asn His Lys Ser Glu Arg Ser Gly Gln His Met Glu Gln Thr Val
                245                 250                 255

Val Arg Lys Gln Phe Val Glu Ser Trp Leu Trp Lys Asn Val Thr Ile
            260                 265                 270

Gly Ser Ser Gly Ser Leu Lys Leu Thr Glu Val Val Pro Asp Thr Thr
        275                 280                 285

Thr Ser Trp Tyr Leu Thr Gly Phe Ser Ile Asp Pro Val Val Gly Leu
    290                 295                 300

Gly Ile Ile Lys Lys Pro Ile Glu Phe Thr Thr Val Gln Pro Phe Phe
305                 310                 315                 320

Ile Val Glu Arg Leu Pro Tyr Ser Ile Lys Arg Gly Glu Ala Ile Glu
                325                 330                 335

Ile Gln Phe Ile Leu Ile Ser Asn Leu Gln Glu Glu His Thr Val Asp
            340                 345                 350

Val Thr Leu Tyr Asn Glu Asn Asn Glu Met Glu Phe Ile Gly Arg Ser
        355                 360                 365

Ile Ala Asn Ala Ser Tyr Thr Lys Ser Val Ser Val Leu Pro Lys Val
    370                 375                 380

Gly Lys Pro Ile Ser Phe Leu Val Lys Ala Lys Lys Leu Gly Glu Met
385                 390                 395                 400

Met Val Arg Val Lys Ala Ser Ile Ala Asn Gly Leu Ala Thr Asp Ala
                405                 410                 415

Leu Glu Lys Val Ile Arg Val Thr Pro Glu Ser Leu Glu Gln Ser Arg
            420                 425                 430

Val Glu Ser Phe Gly Ile Phe Phe Asp Glu Tyr Gln Asn Gln Thr Phe
        435                 440                 445

Ser Val Phe Pro Asn Ile Pro Lys Asn Ala Asp Asn Gly Ser Val Glu
    450                 455                 460

Ile Glu Leu Arg Val Asn Pro Asn Leu Leu Ile Ser Val Lys Glu Asn
465                 470                 475                 480

Leu Asn Asp Ile Arg Thr Asp Trp Ser Glu Cys Ser Lys Gly Ile Arg
                485                 490                 495

Phe Thr Leu Asn Phe Ala Val His Asn Tyr Leu Val Ala Ile Gly Ser
            500                 505                 510

Ser Asp Gln Ile Ser Ser Asp Asp Asn Ala Thr Leu Ser Lys His Tyr
        515                 520                 525

Met Arg Leu Glu Lys Cys Phe Ile Ser Asn Gly Ser Trp Arg Asn Lys
    530                 535                 540

Val Phe Asp Thr Ala Phe Leu Val Asn Ala Leu His Asn Val Ser Ile
545                 550                 555                 560

Tyr Val Ser Trp Lys Asp Lys Arg Lys Leu Glu Lys Ala Phe Asp Trp
                565                 570                 575

Leu Ala Ser Gln Gln His His Ser Gly Ser Phe Lys Glu Thr Glu Ser
            580                 585                 590

Asp Leu His Tyr Lys Arg Ser Asp Val Ala Leu Thr Ser Tyr Val Leu
        595                 600                 605

Ala Val Met Leu Glu Asn Glu Ser Ala Lys Val Glu His Ala Val Val
    610                 615                 620

Ile Glu Lys Gly Met Ser Phe Leu Ser Asp Gln Leu Asp Leu Ile Thr
625                 630                 635                 640

Ser Ala Asn Asp Leu Ala Ile Val Thr Tyr Ala Met Met Leu Tyr Gly
                645                 650                 655

His Arg Leu Lys Asp Ala Ala Phe Glu Lys Leu Ile Asp Met Ser Thr
            660                 665                 670

Ile Thr Asn Asn Gly Thr Glu Arg Tyr Trp Asn Thr Ser Asn Ser Val
        675                 680                 685

Glu Ala Thr Ser Phe Ala Leu Leu Ser His Val Val Ala Asn Lys Leu
    690                 695                 700

Leu Glu Ala Leu Pro Ile Met Arg Trp Leu Val Asn His Arg Ser Glu
705                 710                 715                 720

Leu Asn Ser Val Ser Gly Glu Gln Asn Thr Tyr Leu Arg Phe Lys Ala
                725                 730                 735

Leu Ser Ser Ile Ala Lys Lys Ile Ala Leu Ser Arg Asn Asp Tyr Asp
            740                 745                 750

Val Arg Ile Asn Tyr Arg Leu Arg Arg Glu Leu Phe Arg Phe Thr Ser
        755                 760                 765

Tyr Asp Ser Asp Thr Gln His Met Leu Ile Thr Gln Asn Val Lys Lys
    770                 775                 780

Leu Glu Ile His Val Gly Gly Ile Gly Ala Gly Leu Leu Gln Val Phe
785                 790                 795                 800

Tyr Arg Tyr Asn Leu Asn Leu Met Asn Phe Glu His Arg Phe Lys Leu
                805                 810                 815

Asp Leu Gln Lys Gln Ile Thr Asn Phe Asp Gln Glu Leu Lys Leu Asn
            820                 825                 830

Val Cys Ala Asn Tyr Ile Pro Thr Val Ser Glu Ser Arg Ser Asn Met
        835                 840                 845

Ala Leu Val Glu Val Thr Leu Pro Ser Gly Tyr Val Val Asp His Asn
    850                 855                 860

Pro Ile Ser Glu Gln Thr Thr Val Asn Pro Ile Gln Asn Ile Glu Ile
865                 870                 875                 880

Arg Tyr Gly Gly Thr Ser Val Val Leu Tyr Tyr Asn Ser Met Gly Ser
                885                 890                 895

Glu Arg Asn Cys Phe Thr Val Ser Ala Tyr Arg Arg Phe Lys Val Ala
            900                 905                 910

Leu Lys Arg Pro Ala Tyr Val Val Val Tyr Asp Tyr Tyr Asp Thr Asn
        915                 920                 925

Leu Asn Ala Ile Lys Val Tyr Glu Val Asp Lys Gln Asn Val Cys Glu
    930                 935                 940

Ile Cys Glu Glu Glu Asp Cys Arg Ala Glu Cys Lys Ile
945                 950                 955

## Description

The present invention relates to the prevention of malaria transmission. It focuses on the use of thioester-containing proteins that function in the immune system of the mosquito Anopheles gambiae which is the major vector of human malaria. The invention furthermore relates to a method of making mosquitoes of the genus Anopheles refractory towards the malaria parasite plasmodium, and to refractory mosquitoes produced according to this method.

### Introduction

Malaria is one of the most devastating human diseases, occuring mainly in sub-Saharan Africa. Every year, 300 to 500 million people are infected with the malaria parasite and more than 1 million, mainly children under the age of 5, die as a result (Snow et al.; 1999. Estimating mortality, morbidity and disability due to malaria among Africa's non-pregnant population. Bull World Health Organ 77, 624-640). The main elements in the transmission of malaria are the causative agent, Plasmodium falciparum, the insect vector, Anopheles gambiae, humans and the environment. Disruption of any of these can prevent spreading of the disease. Despite the fact that only a few mosquito species transmit the parasite, it has, to date, not been possible to completely eradicate this disease. Historically, the greatest successes came from the use of insecticides or through the destruction of mosquito habitats to decrease or even completely eliminate mosquito populations. Although this has been successful in some areas, the ecological and sanitary consequences for animals and humans are rarely favorable. Moreover mosquitoes became resistant to many insecticides and recolonized their habitats (Greenwood and Mutabingwa, 2002. Malaria in 2002. Nature 415, 670-672). Alternative attempts have focused on the prevention of the disease transmission to humans through vaccination. However, no effective vaccine against malaria has been reported thus far, mainly due to the fact that the parasite changes its antigenic structures so that vaccines become ineffective (Richie and Saul, 2002. Progress and challenges for malaria vaccines. Nature 415, 694-701). Finally, people from malaria-free countries travelling to malaria-endemic regions usually take oral prophylactic drugs. Most of them kill the parasite by targeting its mitochondrial system, food vacuole or apicoplast (Touze et al., 2002. Mechanism of action of antimalarials. Value of combined atovaquone/proguanil. Med Trop However these drugs have several drawbacks: their side effects in humans, cost burden on the economies of the endemic countries, and the fact that the parasite has meanwhile become resistant to many of them (Sachs and Malaney, 2002. The economic and social burden of malaria. Nature 415, 680-685). Therefore, new approaches have to be considered to prevent malaria transmission. Mosquitoes were shown to be able to mount an immune response against the malaria parasite and it has been suggested that, by rendering this immune response more effective, transmission of the parasite can be completely abolished (Alphey et al., 2002. Malaria control with genetically manipulated insect vectors. Science 298, 119-121). This could be achieved by releasing transgenic mosquitoes that are refractory to parasite infection, as it was suggested by Ito et al., 2002. Transgenic anopheline mosquitoes impaired in transmission of a malaria parasite. Nature 417, 452-455. However, the ecological consequences are in that case, very difficult, if not impossible, to foresee. Thus, key advances in that field will probably come from a better understanding of the mosquito immune response against malaria parasite and the design new strategies for boosting the mosquito natural defense mechanisms.

### Plasmodium development in the mosquito, Anopheles gambiae

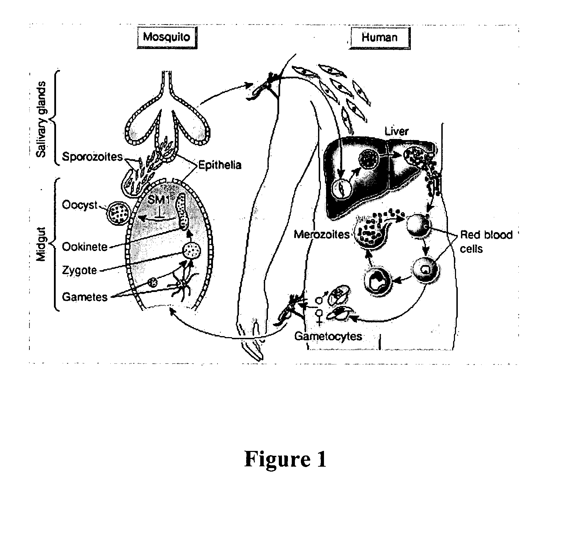

The life cycle of malaria parasite in the mosquito represents a remarkable example of parasite - vector adaptation. Shortly after ingestion of infected blood meal the to male and female parasite gametocytes develop in the mosquito midgut into gametes that undergo fertilization within the next few hours. The newly formed zygotes are progressively transformed into the motile ookinetes that penetrate the peritrophic membrane and the epithelial cells. Once reaching the basal site of the epithelium, the parasite anchors onto the basal membrane and undergoes divisions to produce the oocyst. Some days later the oocyst ruptures and thousands of mature sporozoites are released in the hemocoel. The sporozoite invasion of salivary glands is the last step of the parasite life cycle in the mosquito. After traversing the epithelium the sporozoites reside in the salivary gland lumen, where they further mature before being transferred into a new vertebrate host. In the human, the sporozoites pass through the liver where they are transformed into merozoites that then enter the blood circulation. Within the blood cells, the parasite performs a short, asexual life cycle with many stages (trophozoite, schizont, merozoite). At the end of each cycle, male and female gametocytes are produced. The life cycle of Plasmodium is shown in figure 1.

The major African vector, Anopheles gambiae, mounts immune reactions that differ between genetically selected mosquito strains and account in part for large losses of parasite numbers in the vector. An extreme reaction occurs in a melanotically encapsulating refractory strain, in which late ookinete stage parasites are killed and isolated in a capsule cross-linked with melanin (Collins et al., 1986. Genetic selection of a Plasmodium-refractory strain of the malaria vector Anopheles gambiae. Science 234, 607-610).

### Innate immunity in insects

Insects have been shown to mount an immune response against infections (Hoffmann and Reichhart, 2002. Drosophila innate immunity: an evolutionary perspective. Nat Immunol 3, 121-126). In contrast to chordates, they do not have both innate and adaptive immune systems but rely merely on the most ancient one, the innate immunity. The innate system is the first line of defence in animals and acts quickly at the barrier epithelia, such as epidermis, gut and tracheae, to limit infections. Insects are also able to mount systemic responses, for example, they secrete antimicrobial peptides in their hemolymph (equivalent to vertebrate blood) upon infection.

Thioester-containing proteins (TEPs) have recently been identified in many metazoa, including insects. TEPs from A. gambiae share significant sequence and structural similarities with the complement factors C3/C4/C5, which are known to play an essential role in vertebrate immune responses, and with vertebrate alpha2-macroglobulins (alpha2Ms). TEPs appeared early in evolution: members of this family have been found in such diverse organisms as nematodes, insects, molluscs, fish, birds and mammals (Nonaka, 2000. Origin and evolution of the complement system. Curr Top Microbiol Immunol 248, 37-50). They are characterized by homologous sequence features, including a unique intrachain beta□cysteinyl-gamma□glutamyl thioester, and a propensity for multiple conformationally sensitive binding interactions (Chu et al., 1994. Alpha 2-macroglobulin: a sensor for proteolysis. Ann N Y Acad Sci 737, 291-307). The thioester bond mediates covalent attachment of the molecules to activating self and non-self surfaces (complement factors) and covalent or non-covalent cross-linking to attacking proteases (alpha2Ms). The thioester bond is highly reactive and, to avoid precocious inactivation, it is hidden in the interior of the molecule. Thus, the thioester is exposed only after a proteolytic cleavage of the protein, which is brought about either by a specific "convertase" protease complex, (complement factors), or by attacking proteases (alpha2Ms). The proteolytic activation of the complement factors generates two products, a small anaphylatoxin fragment without the thioester, and a larger fragment that binds covalently to the target surface via the thioester bond. Both cleavage products have signaling properties: anaphylatoxins spread in the vicinity of complement activation and act as chemoattractants to recruit macrophages to the site of infection. Binding of the bigger fragment targets pathogens for phagocytosis or for destruction by lysis, mediated by a membrane attack complex. Alpha2Ms use a different mechanism to inactivate proteases, by denying to the substrates access to the protease active site.

### Characterization of Anopheles TEP1

The cDNA of TEP1 has recently been cloned (Levashina et al., 2001. Conserved Role of a Complement-like Protein in Phagocytosis Revealed by dsRNA Knockout in Cultured Cells of the Mosquito, Anopheles gambiae. Cell 104, 709-718). The amino acid sequence deduced from a cDNA clone codes for a protein of 150 kDa with a signal peptide-like hydrophobic N-terminal segment characteristic of secreted proteins. The sequence contains the canonical thioester motif, followed 100 amino acids downstream by a catalytic histidine residue. The most C-terminal part displays a cysteine signature, characteristic of TEPs found in another insect, the fruitfly Drosophila. The protein is glycosylated and secreted into the hemolymph by mosquito hemocytes; in the hemolymph, it is present as a full-length form of approximately 165 kDa and as a smaller fragment of 80 kDa derived from the C-terminal region (Levashina et al., 2001. Conserved Role of a Complement-like Protein in Phagocytosis Revealed by dsRNA Knockout in Cultured Cells of the Mosquito, Anopheles gambiae. Cell 104, 709-718), indicating a constant low level of proteolytic cleavage of the protein. Interestingly, the same type of cleavage can be induced by both wounding and bacterial challenge, resembling proteolytic activation of complement factors in vertebrates. It is unknown whether cleavage occurs during activation or subsequent inactivation of the molecule.

Mosquito cells in vitro secrete TEP1 into the conditioned medium, where it can be readily detected by affinity-purified rabbit polyclonal antibodies which recognize the full-length and the C-terminal fragment of the molecule. The functionality of the thioester bond in TEP1 is supported by experiments on the denaturation-dependent autocatalytic fragmentation (Levashina et al., 2001. Conserved Role of a Complement-like Protein in Phagocytosis Revealed by dsRNA Knockout in Cultured Cells of the Mosquito, Anopheles gambiae. Cell 104, 709-718). Heat treatment of the conditioned medium leads to the appearance of a new C-terminal fragment of 50 kDa, consistent with the calculated molecular mass of 54 kDa for fragmentation at the thioester motif. Methylamine treatment completely prevents autocatalytic fragmentation (Levashina et al., 2001. Conserved Role of a Complement-like Protein in Phagocytosis Revealed by dsRNA Knockout in Cultured Cells of the Mosquito, Anopheles gambiae. Cell 104, 709-718).

Finally, TEP1 is able to bind to Gram+ and Gram- bacteria in a thioester-dependant manner and this opsonization is essential to promote the phagocytosis of Gram- bacteria by cultured mosquito cells (Levashina et al., 2001. Conserved Role of a Complement-like Protein in Phagocytosis Revealed by dsRNA Knockout in Cultured Cells of the Mosquito, Anopheles gambiae. Cell 104, 709-718) and the uptake of Gram+ and Gram- bacteria by hemocytes in vivo.

### Anopheles TEPs

The recent completion of the Anopheles gambiae genome sequencing (Holt et al., 2002. The genome sequence of the malaria mosquito Anopheles gambiae. Science 298, 129-149) allowed the inventors to search for other members of the TEP family (Christophides et al., 2002. Immunity-related genes and gene families in Anopheles gambiae. Science 298, 159-165). A total of 19 sequences belonging to this family in the mosquito have been identifed, among them 4 genes are likely to be haplotypes, making a total of 15 distinct A. gambiae TEPs. The term "haplotype" usually means (i) a set of alleles borne on one of a pair of homologous chromosomes. (ii) In this context, however, haplotypes are polymorphic sequence stretches, which were different enough not to be assembled in the same contig during the automatic genome assembly. This is due to the discovered particularity of the *A. gambiae* genome which consists of two or more chromosomic forms or haplotypes (i) displaying significant polymorphic differences.

None of the attempts to fight malaria mentioned above have so far been particularly successful. Accordingly it has been an object of the present invention to provide for new ways of making the vector for Plasmodium, i. e. the mosquito Anopheles gambiae, more refractory to this parasite. Furthermore it has been an object of the present invention to provide for new means of interfering with the distribution of the malaria parasite.

All these objects are solved by the use of a thioester-containing protein (TEP) for the manufacture of a medicament for modifying/triggering an immune response in a mosquito of the genus Anopheles against Plasmodium.

The objects of the present invention are also solved by the use of a thioester-containing protein (TEP) for modifying/triggering an immune response in a mosquito of the genus Anopheles against Plasmodium.

In one embodiment, the protein is encoded by a nucleic acid which forms part of the genome of the mosquito of the genus Anopheles.

In one embodiment, the activity of the protein is enhanced or suppressed or induced by application by a compound that interferes with the expression of the protein and/or the activity of the protein, wherein, preferably, the interference with the expression occurs at the transcriptional and/or translational level.

In one embodiment, the compound is applied to the mosquito by feeding, spraying, injection and/or any combination of the aforementioned methods.

In one embodiment, the protein is applied to the mosquito by feeding, spraying, injection and/or any combination of the aforementioned methods.

In one embodiment, the activity of the protein is enhanced or suppressed by induction, enhancement and/or suppression of the expression of the protein in the mosquito.

In one embodiment, induction or enhancement of the expression of the protein is achieved by placing the nucleic acid encoding the protein under the control of an inducible promoter, wherein, preferably, the inducible promoter is selected from the group comprising strong promoters and gene-specific promoters, wherein, more preferably, the strong promoter is a heat shock promoter, preferably the promoter of heat shock protein 70 of D. melanogaster, and the gene-specific promoter is any of the promoters of TEP of A. gambiae.

In one embodiment, the protein is overexpressed.

In one embodiment, suppression of the expression of the protein is achieved by antisense-RNA, double-stranded RNA, insertion of a transgene into the gene of interest and/or by suppression of gene expression by negative feedback regulation.

In one embodiment, the immune response comprises any of the following, in any combination: binding to Plasmodium, opsonizing Plasmodium, killing Plasmodium, e.g. lysing Plasmodium, melanotically encapsulating Plasmodium and phagocytosing Plasmodium.

In one embodiment, Plasmodium is in a stage selected from the group comprising zygote stage, ookinete stage, oocyst stage, sporozoite stage and any combination thereof, wherein, preferably, Plasmodium is in the zygote stage, ookinete stage and/or oocyst stage.

In one embodiment, the protein is fused to another protein having a detrimental effect on Plasmodium.
Examples of such other other proteins fused to a TEP include another TEP (or several TEPs) of the same or different kind.

In one embodiment, the thioester-containing protein (TEP) is a protein having a sequence or part of a sequence selected from the group comprising SEQ ID NO: 1 - 10, or is a protein which can be generated from a protein having a sequence or a part of a sequence selected from the group comprising SEQ ID NO: 1 - 10, by mutation, insertion or deletion of one or several amino acid residues or the corresponding nucleotides coding for these amino acid residues, or is a protein homologous to a protein having a sequence or a part of a sequence selected from the group comprising SEQ ID NO: 1 - 10.

In one embodiment, the thioester-containing protein (TEP) is not TEP4 (not a protein having a sequence as represented by SEQ ID NO: 4).

In one embodiment, the thioester-containing protein (TEP) is a protein, having a sequence or a part of a sequence selected from the group comprising SEQ ID NO: 1 - 3, and 5 - 10, or is a protein which can be generated from a protein having a sequence or a part of a sequence selected from the group comprising SEQ ID NO: 1 - 3 and 5 - 10, by mutation, insertion or deletion of one or several amino acid residues or the corresponding nucleotides coding for these amino acid residues, or is a protein homologous to a protein having a sequence or a part of a sequence selected from the group comprising SEQ ID NO: 1 - 3 and 5 - 10.

Preferably, the thioester-containing protein (TEP) is a protein having a sequence or part of a sequence selected from the group comprising SEQ ID NO: 1 - 3, or is a protein which can be generated from a protein having a sequence or a part of a sequence selected from the group comprising SEQ ID NO: 1 - 3, by mutation, insertion or deletion of one or several amino acid residues or the corresponding nucleotides coding for these amino acid residues, or is a protein homologous to a protein having a sequence or a part of a sequence selected from the group comprising SEQ ID NO: 1 - 3.

More preferably, the thioester-containing protein (TEP) is a protein having a sequence or a part of a sequence selected from the group comprising SEQ ID NO: 1 - 2, or is a protein which can be generated from a protein having a sequence or a part of a sequence selected from the group comprising SEQ ID NO: 1 - 2, by mutation, insertion or deletion of one or several amino acid residues or the corresponding nucleotides coding for these amino acid residues, or is a protein homologous to a protein having a sequence or a part of a sequence selected from the group comprising SEQ ID NO: 1 - 2.

The objects of the present invention are also solved by the use of a thioester-containing protein (TEP) as defined above, to make a mosquito of the genus Anopheles refractory to Plasmodium.

The objects are furthermore solved by the use of a thioester-containing protein (TEP) as defined above, to make a mosquito of the genus Anopheles susceptible to Plasmodium.

The objects of the present invention are also solved by a method for making a mosquito of the genus Anopheles refractory to Plasmodium by inducing, enhancing and/or suppressing expression of a thioester-containing protein, this protein being as defined above.

The objects of the present invention are also solved by a method for making a mosquito of the genus Anopheles susceptible to Plasmodium by inducing, enhancing and/or suppressing expression of a thioester-containing protein, this protein being as defined above.

In one embodiment, the activity of the protein is enhanced or suppressed or induced by application of a compound that interferes with the expression of the protein and/or the activity of the protein, wherein, preferably, the interference with the expression occurs at the transcriptional and/or the translational level.

In one embodiment, the compound is applied to the mosquito by feeding, spraying, injection and/or any combination of the aforementioned methods.

In one embodiment, induction and/or enhancement of expression of the protein occurs by placing the nucleic acid encoding the protein under the control of an inducible promoter, wherein, preferably, the inducible promoter is selected from the group comprising strong promoters and gene-specific promoters, wherein, more preferably, the strong promoter is a heat shock promoter, preferably the promoter of heat shock protein 70 of D. melanogaster, and the gene-specific promoter is any of the promoters of TEP of A. gambiae.

In one embodiment, the protein is overexpressed.

In one embodiment, expression of the protein is knocked out.

Preferably, suppression of the expression of the protein is achieved by antisense-RNA, double-stranded RNA, insertion of a transgene into the gene of interest or by suppression of gene expression by negative feedback regulation.

In one embodiment, the protein is fused to another protein having a detrimental effect on plasmodium.

The objects of the present invention are also solved by a mosquito produced by the method according to the present invention.

It is to be understood that the thioester-containing protein (TEP) according to the present invention may form a complex with other binding partners. These respective binding partners might e.g. be proteins or carbohydrates or glycosylated proteins etc. and can be determined by standard methods available to someone skilled in the art, such as yeast-two-hybrid-systems, surface plasmon resonance, gel filtration, band-shift assays, pull-down assays, analytical ultracentrifugation, NMR etc.

All the objects of the present invention are also solved by a method for identifying compounds that modify/trigger an immune response in a mosquito of the genus Anopheles against Plasmodium comprising the following steps:
a) providing a mosquito that is susceptible to infection by Plasmodium, wherein the susceptibility is caused by a lack or expression of a thioester-containing protein (TEP),
b) exposing the mosquito to a candidate compound suspected of interfering with the activity or the expression of the thioester-containing protein,
c) exposing the mosquito to Plasmodium,
d) determining whether the mosquito has mounted an immune response against Plasmodium.
   Preferably, the immune response of the mosquito is increased by step b).
   In another embodiment, the immune response of the mosquito is decreased by step b).
   In one embodiment, the method comprises the further step:
e) quantifying the immune response against Plasmodium.

The objects of the present invention are also solved by a compound identified by the method according to the present invention, as just described.

In one embodiment, this compound is optimised with respect to its capability of binding to the thioester-containing protein and/or to the nucleic acid(s) encoding the thioester-containing protein.

The objects of the present invention are also solved by a mosquito of the genus Anopheles wherein expression of the protein TEP1 (either TEP1s or TEP1r) is knocked out. In one embodiment the mosquito is a mosquito where TEP1r has been knocked out, preferably by injection of double-stranded RNA (dsRNA) directed against TEP1. Such a mosquito (dsTEP1r) was found to completely have lost its refractory phenotype and has converted into a susceptible mosquito.

In another embodiment the mosquito is a mosquito wherein TEP1s has been knocked out, preferably by overexpressing the gene for TEP1s under the control of a heat shock promoter, more preferably the heat shock promoter 70 of D. melanogaster. Such a hsTP1s transgenic mosquito is likely to behave as a highly susceptible mosquito upon parasite infection, which can for example be used to produce high numbers of parasites. It is clear to someone skilled in the art, that the respective protein sequences for TEP1s and TEP1r (SEQ ID NO: 1 and 2), may be encoded by a number of nucleotide sequences due to the degeneracy of the genetic code. Furthermore the above described knock-out-lines may be lines wherein the above described protein sequences (SEQ ID NO: 1 - 10, preferably SEQ ID NO: 1 - 2) have been altered by mutation, insertion or deletion of one or several amino acid residues or the corresponding nucleotides coding for these amino acid residues. Likewise homologous proteins to the above-mentioned proteins (SEQ ID NO: 1 - 10, preferably 1 - 2) are envisioned.

As used herein the term "homologous" when used in connection with two proteins is meant to signify that the two proteins have evolved from a common ancestor. Preferably the term implies a similarity between two proteins, wherein at least 40 %, preferably at least 60%, more preferably at least 80%, most preferably at least 90% of all amino acid residues in the sequence of one protein are identical or conservatively changed in comparison to the other protein.

A "conservative change" in this context is meant to characterize the mutation from one amino acid residue to another residue that, chemically, belongs to the same group (hydrophobic, charged, etc.). For example a change from Val to Leu would be a conservative change, a change from Val to Glu would not.

The objects of the present invention are also solved by a method of making a transgenic knock-out-line, preferably a transgenic mosquito knock-out-line, more preferably of a mosquito of the genus Anopheles, wherein the gene to be knocked out is placed under the control of an inducible promoter, preferably a heat shock promoter, more preferably the heat shock promoter 70 of D. melanogaster.

The objects of the present invention are also solved by a transgenic knock-out-line produced by a method according to the present invention just described.

The objects of the present invention are also solved by a method of making a transgenic knock-out-line, preferably a transgenic mosquito knock-out-line, more preferably of a mosquito of the genus Anopheles, wherein the gene to be knocked out is placed under the control of an inducible promoter, preferably a heat shock promoter, more preferably the heat shock promoter 70 of D. melanogaster and wherein the expression or overexpression of the gene is induced. Preferably, overexpression is induced. Preferably the overexpression of the gene is induced by rearing a host of the vector, preferably an animal, more preferably a mosquito at 20°C - 45°C, preferably 20°C - 39°C, and more preferably at 20°C - 28°C. Excellent results are obtained by rearing at 20°C for 5 days. Without wishing to be bound by any particular mechanistic theory, an attempt to explain this "knock-out by overexpression" is made below in example 5.

The objects of the present invention are also solved by a nucleic acid vector containing an inducible promoter, preferably a heat shock promoter, and a gene under the control of the inducible promoter.

As described above, TEP1 is involved in the mosquito response to bacterial infection. Moreover, TEP4 was shown to be transcriptionally upregulated upon Plasmodium infection (Oduol et al., 2000. Genes identified by an expression screen of the vector mosquito Anopheles gambiae display differential molecular immune response to malaria parasites and bacteria. Proc Natl Acad Sci USA 97, 11397-11402). However, this on itself, does not constitute a proof that these two proteins are involved in the anti-parasitic response, neither does it give indication on the putative mechanism involved.

The results obtained by the inventors and described below evidence that TEPs play an essential role in anti-parasitic immunity via opsonization and killing of Plasmodium parasites. Their findings provide a method for efficient killing of malaria parasites invading the midgut of the mosquito, as well as, potentially, indications for treatments in humans. Furthermore, the inventors have shown that one allelic form of TEP1 is specific to mosquitoes that are refractory to parasite infection, and this phenomenon might also be true for the allelic forms of three additional TEPs. These newly discovered TEP variants open a wide range of possible ways to prevent Plasmodium transmission and to study Plasmodium development, which is explained in more detail below.

The inventors have surprisingly found that TEPs play a paramount role in the immune response that the mosquito vector mounts, once the parasite has entered their midgut. The idea is that, by selectively influencing the activity or the expression levels of any of the proteins or protein complexes that involve any of these proteins according to the present invention, the immune response of the mosquito can be altered so as to increase or decrease the number of parasites produced, as required or desired. There are many ways of influencing the activity and the expression levels of the aforementioned proteins. This can be done for example, by treatment of *Anopheles* by either feeding, spraying or injection of small molecules that interfere with the proteins or the protein complexes enhancing or suppressing their activity, or that interfere with their expresion. Also, for modification of the protein expression level mosquito knock-out-lines can be produced using antisense-technology or RNA interference technology (RNAi). The principle of knocking out a gene by injecting double stranded RNA (dsRNA) is well known to someone skilled in the art and has since been well established. In brief, it works as follows: Double-stranded RNA corresponding to the targeted gene is produced in vitro by standard transcription methods and is then injected into newly emerged adult mosquitoes, preferably, in concentrations varying from 1 to 5 micrograms per microliter. Silencing of gene expression is checked by standard molecular biology techniques (RT-PCR, Q-PCR, Northern, Western).

Thereby genes of interest can be silenced.

Likewise transgenic methods of inducing/enhancing or silencing the expression of genes of interest are well known to someone skilled in the art. This is generally performed by placing the gene of interest or a transgene producing double-stranded RNA of the gene of interest, respectively, under the control of an inducible promoter, such that by induction of the promoter of the gene can be switched on and its product would be expressed in large amounts.

Although the invention has been described with an emphasis on Anopheles gambiae, it should be stressed that it is not limited thereto:

Malaria is transmitted by several Anopheles species, for example A. albimanus, A. arabiensis, A. funestus, A. gambiae, etc.... The human parasite is Plasmodium genus, however four different species are recorded: P. falciparum, P. vivax, P. ovalae, P. malariae. It is anticipated that the interaction between all Plasmodia and Anopheles vectors are similar, thus the proteins of interest (TEP) are exptected to function similarly in these interactions and their modification could affect transmission of the different Plasmodium by the various species. Although these proteins are not yet identified in species other than A. gambiae and although it is expected that they are somehow diversified, they will most likely follow similar patterns with respect to their roles in the immune response mounted by the respective Anopheles vector and will be homologous thereto.

As used herein, the terms "knock-out" of genes and "silencing" of genes are used interchangeably throughout this application.

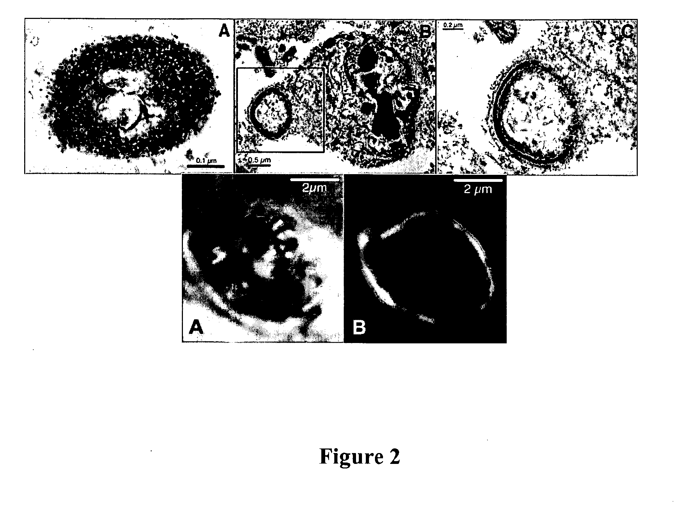

The inventors have shown that Anopheles TEP1 was able to opsonize very different pathogens, such as Gram+ and Gram- bacteria (prokaryotes) (Levashina et al., 2001, ibid.) and Plasmodium (protozoan parasites) (see figure 2) and promote their uptake by hemocytes (in the case of bacteria) (Levashina, ibid.) or their lysis and clearance (in the case of Plasmodium) (this application). It is envisioned that TEPs might also be able to recognize and opsonize other pathogens and trigger the mosquito immune response in relation to these other pathogens. Examples of other pathogens transmitted by mosquitoes are Wucheria and Brugia (filariae, nematodes) which are the causative agents for human filariasis.

Reference is now made to the figures, wherein
figure 1 shows the lifecycle of Plasmodium in the human host and the mosquito vector,
figure 2 shows the opsonizing effect that TEP1 has on bacteria and malaria parasites.
figure 3 shows the result of an RT-PCR experiment showing the specificity of TEP16 (= TEP1r) to refractory mosquitoes, and
figure 4 shows the results of a western blot analysis, showing a knock out of TEP1s placed under the control of a heat shock promoter, and
figure 5 shows an opsonisation event of plasmodium.

Furthermore reference is made to the attached sequence listing, wherein
SEQ ID NO: 1 shows the protein sequence of TEP1 as it is found in the susceptible Anopheles strain (which is the reason it is also called TEP1s),
SEQ ID NO: 2 is the protein sequence of TEP16, which also sometimes is referred to as TEP1r for the reason that it is found in the refractory strain of Anopheles and has a high sequence similarity to TEP1s (~94% identity),
SEQ ID NO: 3 shows the protein sequence of TEP3,
SEQ ID NO: 4 shows the protein sequence of TEP4,
SEQ ID NO: 5 shows the protein sequence of TEP5,
SEQ ID NO: 6 shows the protein sequence of TEP17 which is an allele of TEP5,
SEQ ID NO: 7 shows the protein sequence of TEP6,
SEQ ID NO: 8 shows the protein sequence of TEP 18 which is an allele of TEP6
SEQ ID NO: 9 shows the protein sequence of TEP8, and
SEQ ID NO: 10 shows the protein sequence of TEP19 which is an allele of TEP8.

In more detail, figure 1 shows the lifecycle of the plasmodium parasite, shows *Plasmodium* development in the human host and the mosquito vector (*1*). Shortly after ingestion of infected blood meal, male and female gametocytes develop into gametes in the mosquito midgut that undergo fertilization within the next few hours. The newly formed zygotes are progressively transformed into the motile ookinetes that penetrate the peritrophic membrane and the epithelial cells. Once reaching the basal site of the epithelium, the parasite anchors onto the basal membrane and undergoes divisions to produce the oocyst. Some days later the oocyst ruptures and thousands of mature sporozoites are released in the hemocoel. The sporozoite invasion of salivary glands is the last step of the parasite life cycle in the mosquito. After traversing the epithelium sporozoites reside in the salivary gland lumen, where they further mature before being transferred into a new vertebrate host. In the human, the sporozoites pass through the liver where they are transformed into merozoites that then enter the blood circulation. Within the blood cells, the parasite performs a short, asexual life cycle with many stages (trophozoite, schizont, merozoite). At the end of each cycle, male and female gametocytes are produced.

Figure 2 shows the opsonization of bacteria and malaria parasites by TEP1, in particular the top panel shows transmission electron micrographs showing TEP1 immunogold labeling **(A)** on the surface of a bacterium during natural bacterial infection of the mosquito midgut; **(B)** on the surface of a Plasmodium berghei ookinete 30 hours after the infectious blood meal. **(C)** Detailed micrograph of the apical part of the ookinete from **(B)**. Bottom panel: Confocal micrograph showing binding of TEP1 to the surface of a young P. berghei oocyst 48 hours after the infectious blood meal. **(A)** phase contrast image of the oocyst; **(B)** TEP1 (green) colocalizes (yellow) with the ookinete surface protein P28 (red). Nucleic acids are stained in blue.

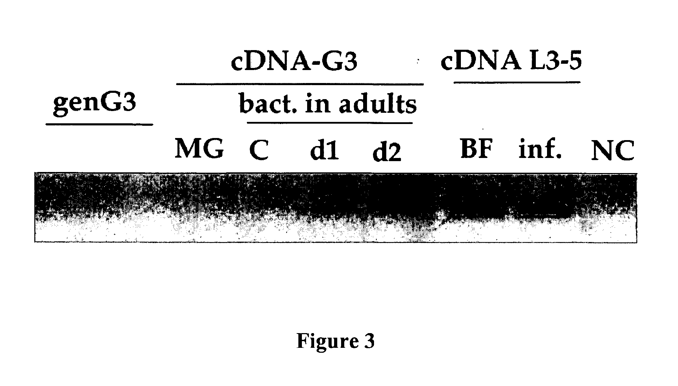

Figure 3 shows results of a Reverse Transcription PCR experiment showing that TEP16 (=TEP1r) is expressed in resistant but not in susceptible mosquitoes (L3-5 and G3 strains respectively). Total RNA was extracted from susceptible adult mosquitoes (G3): midgut only (MG), naïve adults (C) and 1 day (d1) or 2 days (d2) after bacterial challenge. Total RNA was extracted from resistant adult mosquitoes (L3-5): blood-fed (BF) or infected with Plasmodium (inf.). Total RNAs were reverse-transcribed into cDNAs that were then amplified by PCR using TEP16 specific primers. A negative control (NC), without any template, was included.

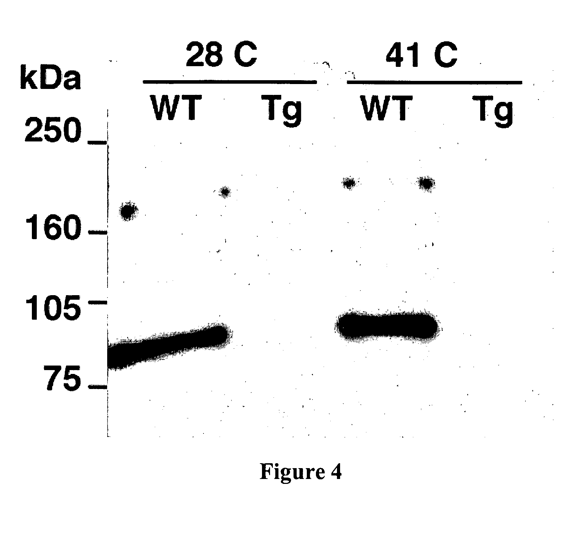

Figure 4 shows that TEP1 expression is silenced in one transgenic line transformed with the hsTEP1s construction. Hemolymph was collected from wild type (WT) and transgenic (Tg) mosquitoes that were either reared in normal conditions (28°C) or heat-shocked at 41°C for 1 hour (41°C) and analyzed by Western Blot using TEP1 specific antibodies. Two forms of the protein are present in the hemolymph of WT mosquitoes: a full-length form at 180 kDa and a predominant processed form (C-terminal part) at 80 kDa. In contrast, no TEP1 was detectable in transgenic mosquitoes.

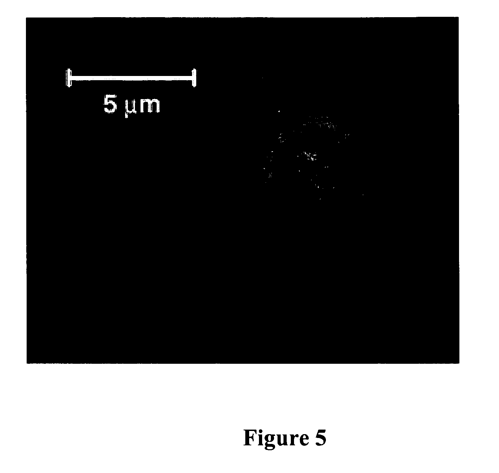

Figure 5 shows that TEP16 (=TEP1r) and PPOs do not colocalize on the surface of Plasmodium in refractory mosquitoes. The confocal micrograph shows that a P. berghei parasite (red, inner circle) opsonized by TEP1r (green, middle circle), is surrounded by a PPO layer (blue, dark circle), itself covered by TEP1r (outer circle), but no colocalization between TEP1r and PPOs is detectable.

In the following the invention will be described in greater detail by reference to the following examples which are given to illustrate, not to limit the invention.

### EXAMPLES

### Example 1

### Opsonization of malaria parasites by TEP1

The inventors have found TEP1 on the surface of the parasites, when the ookinetes complete their passage through the midgut cells and reach the basal lamina (see Figure 2). This binding is specific to the midgut parasite stages, ookinete and oocyst, which are separated from the mosquito hemocoel by a porous noncellular barrier, the fibrous basal lamina. Later parasite stages, sporozoites, which have to cross the hemocoel on their way to the salivary glands, are not recognized by TEP1. Thus, binding of TEP1 to the parasite surface means that this molecule recognizes the parasite and signals to activate an immune response.

### Example 2

### Characterization of parasite development in susceptible mosquitoes deficient for TEP1

Infection of TEP1-deficient mosquitoes with Plasmodium parasites leads to a higher number of parasite oocysts on the mosquito midgut. To silence TEP1 gene expression, the inventors injected dsRNA directed against TEP1, into the thorax of young females This technique is described in Blandin et al., 2002, EMBO rep. 3, No. 9, 852-856 and works as follows: the dsRNA is taken up by cells and cleaved into small interfering fragments that trigger the specific degradation of the endogenous target mRNA. Four days later, the mosquitoes thus treated were fed on P. berghei-infected mice. The mosquito midguts were dissected and the number of developed oocysts was counted ten days after the infectious bloodmeal. A five-fold increase in the mean number of oocysts on dsTEP1 midguts as compared to dsGFP controls (i. e. mosquitoes that had been injected with double stranded RNA for green fluorescent protein (GFP)) was observed. Moreover, three weeks post-infection, dsTEP1-treated mosquitoes contained higher numbers of sporozoites in the salivary glands than the control mosquitoes. These results clearly demonstrate for the first time, that mosquitoes are indeed mounting an effective immune response against the malaria parasites, predominantly at the critical moment of parasite development, before the ookinete becomes an oocyst.

Thus, the described functional studies and the specific opsonization of Plasmodium midgut stages (ookinetes, oocysts, but not sporozoites, see above) by TEP1 mean that TEP1 plays a key role in the recognition and killing of the midgut stages of malaria parasite in mosquito. In TEP1-deficient mosquitoes, oocysts are not killed during the midgut invasion ultimately resulting in an increased number of sporozoites in the salivary glands.

### Example 3

### TEP16 is an allelic form of TEP1, specific to refractory mosquitoes

One of the putative genes identified through the analysis of the whole mosquito genome sequence and named TEP16, was highly homologous to TEP1. Using RT-PCR, the inventors have shown that TEP16 was present only in the genome of refractory mosquitoes but not in susceptible mosquitoes, indicating that TEP 16 represents an allele of TEP 1, now called TEP1r (see fig. 3). Furthermore, the TEP1 locus is located in the Pen 2 (Plasmodium encapsulation 2) region, one out of three loci shown to be essential for the encapsulation phenotype of refractory mosquitoes. Importantly, genetic analysis links TEP1 with the markers specific for the refractory strain (Zheng et al., 1997. Quantitative trait loci for refractoriness of Anopheles gambiae to Plasmodium cynomolgi B. Science 276, 425-428). Of special interest is the structure-functional comparative analysis between two forms of TEP1 that will be instructive to a person skilled in heart in defining mechanisms of specificity of recognition and of efficiency of parasite killing mediated by TEP1. Noteworthy, the differences between TEP1s/r are not homogenously scattered along the molecule. Instead, clusters are found in the central region (around the thioester, likely to be involved in opsonization preferences), as well as in the C-terminal domain (covalently bound to pathogens via the thioester and probably recruiting killing molecules or involved in signalling), suggesting that both recognition and signalling efficiency are affected by the allelic differences.

### Example 4

### Characterization of parasite development in refractory mosquitoes deficient for TEP1r

The inventors have extended their analysis to the more specific question whether TEP1 is involved in melanotic refractoriness. For this, they used the refractory L3-5 strain of A. gambiae (Collins et al., 1986. Genetic selection of a Plasmodium-refractory strain of the malaria vector Anopheles gambiae. Science 234, 607-610), which does not support parasite development and melanizes ookinetes. Importantly, melanization occurs at the basal side of the mosquito midgut, at the very place where TEP1 is observed to bind to the ookinetes in susceptible mosquitoes. The dsTEP1 knockout (as described above in Example 2) completely abolishes the refractory phenotype and converts refractory mosquitoes into susceptible.

It is likely that TEP1 does not directly anchor a prophenoloxidase (PPO) or a PPO-activating complex on the surface of the parasite. Indeed, the parasites, when opsonized by TEP1, are surrounded by a thick layer of PPO(s), which, at later stages, is itself surrounded by a second layer of TEP1, but no co-localization of the two proteins was observed (see figure 5).

The refractory phenotype most likely represents a combination of two independent events, parasites are first recognized and killed by TEP1 and then melanized in TEP1-independent manner. If so, one should find on the midgut of refractory mosquitoes parasites that are killed but not yet melanized. To show this, the inventors made use of transgenic parasites that express GFP under the promoter of elongation factor 1 (P{elF1}::GFP) and that were shown to lose GFP fluorescence shortly after death. The parasites that were opsonized with TEP1 did not express GFP any longer but were not yet melanized. These parasites were smaller in size than GFP-positive parasites, contained compact nuclei, and often no nuclei was detectable by DAPI nucleostaining. These results taken together confirm that TEP1 is essential for parasite killing in refractory as well as in susceptible mosquitoes and that melanization of parasites in the refractory strain occurs after the killing, probably to isolate dead parasites from the mosquito midgut.

### Example 5

### Determination of the role of TEP1s/r and of their thioester site in the mosquito anti-parasitic response

Overexpression of TEP1s in susceptible strains should lead to more efficient killing of the parasite. Surprisingly, it was found that a transgenic line overexpressing TEP1s under the Drosophila promoter of the heat shock protein 70 (hsTEP1s), was showing a knock out phenotype in adults reared at 28°C throughout, or 41°C for 1 hour (see fig. 4). Similar results can be obtained by rearing at 37°C for 1 day. TEP1 expression was decreased both at the transcriptional and protein levels. This knock out might be due to a mechanism similar to RNA interference, to a tight regulation of TEP1 expression or to the disruption of TEP1 gene or promoter by the transgene. This is the first example of "knock out by overexpression" phenotype in insects, thus far post-transcriptional gene silencing associated by a transgene expression has been described only in plants (Metzlaff, 2002) and in C. elegans (Dernburg et al., 2000; Ketting and Plasterk, 2000). The vector generated here could be used generally to create knock out phenotypes in the mosquito.

The hsTEP1s transgenic mosquitoes behave as highly susceptible mosquitoes upon parasite infection, similar to the dsTEP1 knock out mosquitoes generated by dsRNA injection, which provides a useful tool to generate numerous oocyst and sporozoite stages. So far, the analysis of parasite transcriptional and protein profiles was hampered by the low parasite numbers in mosquitoes, especially during these two stages. Higher parasite numbers in the fully susceptible mosquitoes will allow to overcome this caveat. The molecular analysis of the parasite developmental stages will be instrumental for the design of intervention measures and could lead to development of a new generation of drugs and vaccines.

### Example 6

### Proposed function for TEP1 in the mosquito immune response against malaria parasites and perspectives

Taking into account all previously described results, but without wishing to be bound by a particular mechanistic theory the inventors believe the following: TEP1 is able to recognize and opsonize Plasmodium in susceptible and refractory mosquitoes as they are traversing the midgut epithelium to reach the basal side. Under the basal lamina, parasites come in contact with the mosquito hemolymph and its components, but are protected from direct contact with the hemocytes. TEP1 being present in the hemolymph recognizes and binds to the parasite surface. This labeling targets parasites for killing via as yet unknown mechanism. Should TEP1 behave as complement-like factors, the covalent binding of its C-terminal part could recruit the formation of a membrane attack complex (that could be constituted by other TEPs) and allow for the parasite lysis. An important difference between susceptible and refractory mosquitoes is that all the parasites are killed in the refractory strain, but not in the susceptible, suggesting that TEP1r might be more efficient in labeling or/and assembly of the killing complex than TEP1s. The second important difference between the two strains pertains to the parasite clearance after the killing: in the refractory strain the killed parasites are isolated from mosquito tissues by a melanin capsule, whereas in susceptible strain parasites are, most probably, completely lysed. The N-terminal part of proteolytically activated TEP1 could be involved in further signalling to the nearby hemocytes. Thus the differences between TEP1s and TEP1r forms might explain both phenotypes: more efficient parasite opsonization/killing in the refractory strain and distinct processes of clearance (lysis versus melanotic encapsulation) observed in these two mosquito strains.

One could think of using TEP1r or parts of its sequence fused to "killer factors" such as toxic small molecules, enzymes, immune recognition factors which would lead to an even more efficient killing of the parasite.

### Example 7

### Role of TEP3 and TEP4 in the mosquito immune response

Although the expression of TEP4 was reported to be upregulated upon parasite infection (Oduol 2000), the inventors report preliminary experiments showing that the silencing of TEP4 has no effect on parasite survival. However, TEP4-deficient mosquitoes are susceptible or highly susceptible to infections with Gram- or Gram+ bacteria respectively, suggesting that TEP4 would rather play a role in the immune response against bacteria and might not be involved in the anti-parasitic response. TEP3-deficient mosquitoes are more susceptible to both Gram+ and Gram- bacterial infections than controls, however to a lesser extent than TEP4-deficient mosquitoes. In contrast to TEP4 (SEQ ID NO: 4), TEP3 (SEQ ID NO: 3) seems also to be involved in the immune response against Plasmodium parasites as its silencing allows more parasites to develop on the mosquito midgut. This is also suggested by the locus to which TEP3 maps which is Pen2, i. e. the same locus as TEP1. These results suggest that TEP3 might be one of the effector molecules recruited by TEP1 after the recognition and opsonization of pathogens.

### Role of other TEPs in the mosquito immune response

As described in (Christophides et al., 2002), the comparison of TEP sequences from A. gambiae, D. melanogaster and C. elegans, shows that all the mosquito TEPs located on the left arm of the third chromosome (TEP1, 3-11) cluster in an Anopheles-specific expansion and have no orthologues in the fruitfly. This suggests that these mosquito TEPs have evolved to recognize and kill the pathogens specifically encountered by the mosquito (which life style is quite different from the fruitfly) and, especially, Plasmodium parasites.

As well as for TEP1, allelic forms for TEP5, 6 and 8 (SEQ ID NO: 5, 7 and 9) have been identified (TEP 17, 18 and 19 respectively) (SEQ ID NO: 6, 8 and 10). All these TEPs are located within the same genetic region as TEP1 (Pen 2). These observations put forward for consideration the following hypothesis: allelic forms of TEP5, 6 and 8 might also be specific to the refractory mosquito strain and, if this is true, then TEP 16, 17, 18 and 19 might be acting in concert to recognize and kill all Plasmodium parasites and target them for clearance via melanization.

### LITERATURE CITED

Alphey, L. et al. (2002). Malaria control with genetically manipulated insect vectors. Science 298, 119-121.
Christophides, et al. (2002). Immunity-related genes and gene families in Anopheles gambiae. Science 298, 159-165.
Chu, C. T., et al. (1994). Alpha 2-macroglobulin: a sensor for proteolysis. Ann N Y Acad Sci 737, 291-307.
Collins, F. H., et al. (1986). Genetic selection of a Plasmodium-refractory strain of the malaria vector Anopheles gambiae. Science 234, 607-610.
Dernburg, A. F., et al. (2000). Transgene-mediated cosuppression in the C. elegans germ line. Genes Dev 14, 1578-1583.
Greenwood, B., et al. (2002). Malaria in 2002. Nature 415, 670-672.
Hoffmann, J. A., et al. (2002). Drosophila innate immunity: an evolutionary perspective. Nat Immunol 3, 121-126.
Holt, R. A., et al. (2002). The genome sequence of the malaria mosquito Anopheles gambiae. Science 298, 129-149.
Ito, et al. (2002). Transgenic anopheline mosquitoes impaired in transmission of a malaria parasite. Nature 417, 452-455.
Ketting, R. F., et al. (2000). A genetic link between co-suppression and RNA interference in C. elegans. Nature 404, 296-298.
Levashina, E. A., et al. (2001). Conserved Role of a Complement-like Protein in Phagocytosis Revealed by dsRNA Knockout in Cultured Cells of the Mosquito, Anopheles gambiae. Cell 104, 709-718.
Metzlaff, M. (2002). RNA-mediated RNA degradation in transgene- and virus-induced gene silencing. Biol Chem 383, 1483-1489.
Nonaka, M. (2000). Origin and evolution of the complement system. Curr Top Microbiol Immunol 248, 37-50.
Oduol, F., et al. (2000). Genes identified by an expression screen of the vector mosquito Anopheles gambiae display differential molecular immune response to malaria parasites and bacteria. Proc Natl Acad Sci U S A 97, 11397-11402.
Richie, T. L., et al. (2002). Progress and challenges for malaria vaccines. Nature 415, 694-701.
Sachs, J., et al. (2002). The economic and social burden of malaria. Nature 415, 680-685.
Snow, R. W., et al. (1999). Estimating mortality, morbidity and disability due to malaria among Africa's non-pregnant population. Bull World Health Organ 77, 624-640.
Touze, J. E., et al. (2002). Mechanism of action of antimalarials. Value of combined atovaquone/proguanil. Med Trop (Mars) 62, 219-224.
Zheng, L., et al. (1997). Quantitative trait loci for refractoriness of Anopheles gambiae to Plasmodium cynomolgi B. Science 276, 425-428.
The features of the present invention disclosed in the specification, the claims, the sequence listing and/or in the accompanying drawings, may, both separately, and in any combination thereof, be material for realising the invention in various forms thereof.

## Claims

1. Use of a thioester-containing protein (TEP) for the manufacture of a medicament for modifying/triggering an immune response in a mosquito of the genus Anopheles against Plasmodium.

2. Use according to claim 1, wherein the protein is encoded by a nucleic acid which forms part of the genome of the mosquito of the genus Anopheles.

3. Use according to any of claims 1 - 2, wherein the activity of the protein is enhanced or suppressed or induced by application by a compound that interferes with the expression of the protein and/or the activity of the protein.

4. Use according to claim 3, wherein the interference with the expression occurs at the transcriptional and/or translational level.

5. Use according to any of claims 3 - 4, wherein the compound is applied to the mosquito by feeding, spraying, injection and/or any combination of the aforementioned methods.

6. Use according to any of the claims 1 - 2, wherein the protein is applied to the mosquito by feeding, spraying, injection and/or any combination of the aforementioned methods.

7. Use according to any of claims 1 - 2, wherein the activity of the protein is enhanced or suppressed by induction, enhancement and/or suppression of the expression of the protein in the mosquito.

8. Use according to claim 7, wherein induction or enhancement of the expression of the protein is achieved by placing the nucleic acid encoding the protein under the control of an inducible promoter.

9. Use according to claim 8, wherein the inducible promoter is selected from the group comprising strong promoters and gene-specific promoters.

10. Use according to claim 9, wherein the strong promoter is a heat shock promoter, preferably the promoter of heat shock protein 70 of D. melanogaster, and the gene-specific promoter is any of the promoters of TEP of A. gambiae.

11. Use according to any of claims 7 - 10, wherein the protein is overexpressed.

12. Use according to claim 7, wherein suppression of the expression of the protein is achieved by antisense-RNA, double-stranded RNA, insertion of a transgene into the gene of interest and/or by suppression of gene expression by negative feedback regulation.

13. Use according to any of the foregoing claims, wherein the immune response comprises any of the following, in any combination: binding to Plasmodium, opsonizing Plasmodium, killing Plasmodium, e.g. lysing Plasmodium, melanotically encapsulating Plasmodium and phagocytosing Plasmodium.

14. Use according to any of the foregoing claims, wherein Plasmodium is in a stage selected from the group comprising zygote stage, ookinete stage, oocyst stage, sporozoite stage and any combination thereof.

15. Use according to claim 14, wherein Plasmodium is in the zygote stage, ookinete stage and/or oocyst stage.

16. Use according to any of the foregoing claims, wherein the protein is fused to another protein having a detrimental effect on Plasmodium.

17. Use according to any of the foregoing claims, wherein the thioester-containing protein (TEP) is a protein having a sequence or part of a sequence selected from the group comprising SEQ ID NO: 1 - 10, or is a protein which can be generated from a protein having a sequence or a part of a sequence selected from the group comprising SEQ ID NO: 1 - 10, by mutation, insertion or deletion of one or several amino acid residues or the corresponding nucleotides coding for these amino acid residues, or is a protein homologous to a protein having a sequence or a part of a sequence selected from the group comprising SEQ ID NO: 1 - 10.

18. Use according to any of the foregoing claims, wherein the thioester-containing protein (TEP) is not TEP4 (not a protein having a sequence as represented by SEQ ID NO: 4).

19. Use according to any of the foregoing claims, wherein the thioester-containing protein (TEP) is a protein, having a sequence or a part of a sequence selected from the group comprising SEQ ID NO: 1 - 3, and 5 - 10, or is a protein which can be generated from a protein having a sequence or a part of a sequence selected from the group comprising SEQ ID NO: 1 - 3 and 5 - 10, by mutation, insertion or deletion of one or several amino acid residues or the corresponding nucleotides coding for these amino acid residues, or is a protein homologous to a protein having a sequence or a part of a sequence selected from the group comprising SEQ ID NO: 1 - 3 and 5 - 10.

20. Use according to any of the foregoing claims, wherein the thioester-containing protein (TEP) is a protein having a sequence or part of a sequence selected from the group comprising SEQ ID NO: 1 - 3, or is a protein which can be generated from a protein having a sequence or a part of a sequence selected from the group comprising SEQ ID NO: 1 - 3, by mutation, insertion or deletion of one or several amino acid residues or the corresponding nucleotides coding for these amino acid residues, or is a protein homologous to a protein having a sequence or a part of a sequence selected from the group comprising SEQ ID NO: 1 - 3.

21. Use according to claim 20, wherein the thioester-containing protein (TEP) is a protein having a sequence or a part of a sequence selected from the group comprising SEQ ID NO: 1 - 2, or is a protein which can be generated from a protein having a sequence or a part of a sequence selected from the group comprising SEQ ID NO: 1 - 2, by mutation, insertion or deletion of one or several amino acid residues or the corresponding nucleotides coding for these amino acid residues, or is a protein homologous to a protein having a sequence or a part of a sequence selected from the group comprising SEQ ID NO: 1 - 2.

22. Use of a thioester-containing protein (TEP) as defined in any of the foregoing claims, to make a mosquito of the genus Anopheles refractory to Plasmodium.

23. A method for making a mosquito of the genus Anopheles refractory to Plasmodium by inducing, enhancing and/or suppressing expression of a thioester-containing protein, this protein being as defined in any of the foregoing claims.

24. The method according to claim 23, wherein the activity of the protein is enhanced or suppressed or induced by application of a compound that interferes with the expression of the protein and/or the activity of the protein.

25. The method according to claim 24, wherein the interference with the expression occurs at the transcriptional and/or the translational level.

26. The method according to any of claims 24 - 25, wherein the compound is applied to the mosquito by feeding, spraying, injection and/or any combination of the aforementioned methods.

27. The method according to claim 23, wherein induction and/or enhancement of expression of the protein occurs by placing the nucleic acid encoding the protein under the control of an inducible promoter.

28. The method according to claim 27, wherein the inducible promoter is selected from the group comprising strong promoters and gene-specific promoters.

29. The method according to claim 28, wherein the strong promoter is a heat shock promoter, preferably the promoter of heat shock protein 70 of D. melanogaster, and the gene-specific promoter is any of the promoters of TEP of A. gambiae.

30. The method according to claim 29, wherein the protein is overexpressed.

31. The method according to claim 29, wherein expression of the protein is knocked out.

32. The method according to claim 23, wherein suppression of the expression of the protein is achieved by antisense-RNA, double-stranded RNA, insertion of a transgene into the gene of interest or by suppression of gene expression by negative feedback regulation.

33. The method according to any of claims 23 - 32, wherein the protein is fused to another protein having a detrimental effect on plasmodium.

34. A mosquito produced by the method according to any of claims 23 - 33.
